## Europäisches Patentamt

(19) **European Patent Office**

**Office européen des brevets**

(11) Publication number: **0 102 634**
**B1**

(12) # EUROPEAN PATENT SPECIFICATION

(45) Date of publication of patent specification: **29.06.88**

(21) Application number: **83108699.6**

(22) Date of filing: **03.09.83**

(51) Int. Cl.⁴: **C 12 N 15/00,** C 12 P 21/00,
C 12 P 21/04, C 12 P 19/34,
A 61 K 39/395 // C12R1/19

(54) Recombinant DNA, microorganism transformed therewith and use thereof.

(30) Priority: **07.09.82 JP 156285/82**

(43) Date of publication of application:
**14.03.84 Bulletin 84/11**

(45) Publication of the grant of the patent:
**29.06.88 Bulletin 88/26**

(84) Designated Contracting States:
**AT BE CH DE FR GB IT LI NL SE**

(56) References cited:

PATENT ABSTRACTS OF JAPAN, unexamined
application, section C, vol. 7, no. 97, April 23,
1983 THE PATENT OFFICE JAPANESE
GOVERNMENT page 50 C 163 (1242)

(73) Proprietor: **Takeda Chemical Industries, Ltd.**
**27, Doshomachi 2-chome Higashi-ku**
**Osaka-shi Osaka, 541 (JP)**

(72) Inventor: **Kikuchi, Masakazu**
**4-16, Higashitokiwadai 7-chome Toyono-cho**
**Toyono-gun Osaka 563-01 (JP)**
Inventor: **Kurokawa, Tsutomu**
**1-50, Suideidai 1-chome**
**Kawanishi Hyogo 666-01 (JP)**
Inventor: **Onda, Haruo**
**21-6, Aza-junmatsu Tada-in**
**Kawanishi Hyogo 666-01 (JP)**

(74) Representative: **von Kreisler, Alek, Dipl.-Chem.**
**et al**
**Deichmannhaus am Hauptbahnhof**
**D-5000 Köln 1 (DE)**

Courier Press, Leamington Spa, England.

# 0 102 634

**Description**

This invention relates to a novel DNA. More particularly, it relates to a DNA containing a polynucleotide which codes for the human immunoglobulin E H-chain polypeptide, to a transformant carrying said DNA, and to a method for producing the human immunoglobulin E H-chain polypeptide by the cultivation of said transformant.

Immunoglobulins, which are present in animal body fluids and are closely associated with antibodies, consist of H (heavy) chains and L (light) chains. Each chain comprises the V region, which is determinative of the binding specificity with antigen, and the C region, which is determinative of the biological function. On the basis of the constituents of the H chains, immunoglobulins (Ig) are classified into 5 classes, namely A, D, G, M and E.

Among them, immunoglobulin E (hereinafter referred to as IgE), which constitutes reagin, has a molecular weight of 196,000 daltons and consists of two 75,000-dalton H chains and two 22,500-dalton L chains (in the case of human IgE), the chains being linked together by disulfide bond. The C region of the H chain of IgE comprises four sites, CH1 to CH4, and two H chains are linked together at CH2 by disulfide bonds. IgE is in charge of important biological reactions, such as allergic reactions. For instance, it is known that allergic reactions are induced by binding of specific antigen-bound IgE to sensitized mast cells or basophilic cells [K. Ishizaka and T. Ishizaka, Immunological Rev., 41, 109 (1978)]. Therefore, for the purpose of suppressing allergic reactions, the use of an IgE molecule having no antigen-binding site has been proposed. However, many problems remain unsolved with respect to a variety of *in vivo* reactions induced by IgE. One reason is that a sufficient quantity of human IgE cannot be supplied.

On the other hand, the anti-IgE antibody is an essential material in the diagnosis of allergic diseases and is demanded in very large quantity. For its production, however, the purified human IgE is required in large quantity. For this and other reasons, development of a technique capable of producing the human IgE on large scale and at low cost has been waited for.

In a so-far proposed method of producing IgE, the supernatant of a culture of human IgE-producing myeloma cells of an established line is treated for the separation of IgE followed by purification. However, said method involves cell culture and the cell growth rate is low. For these and other reasons, it is difficult to obtain a large quantity of IgE at low cost.

The present inventors continued their researches, with use of the technology of gene manipulation, on the basis of the human IgE-encoding mRNA isolated from cells [Japanese Patent Application No. 120,555/1981 filed on July 30, 1981: Japanese Patent Unexamined Publication (Kokai) No. 21,693/1983] so that they could develop a technology of producing the human IgE H chain polypeptide by cloning the gene coding for the human IgE H chain polypeptide and introducing the thus-obtained recombinant DNA molecule into a host organism, and, as a result, they have completed the present invention.

Thus, the present invention provides a DNA which contains a polynucleotide coding for the human IgE H-chain polypeptide, a transformant carrying said DNA, and a method of producing the human IgE H-chain polypeptide or a polypeptide equivalent thereto in immunological or biological activities, which comprises growing the transformant carrying said DNA.

The DNA provided by the present invention is a DNA containing a polynucleotide having the nucleotide sequence shown in Figure (hereinafter referred to as Fig.) 1.

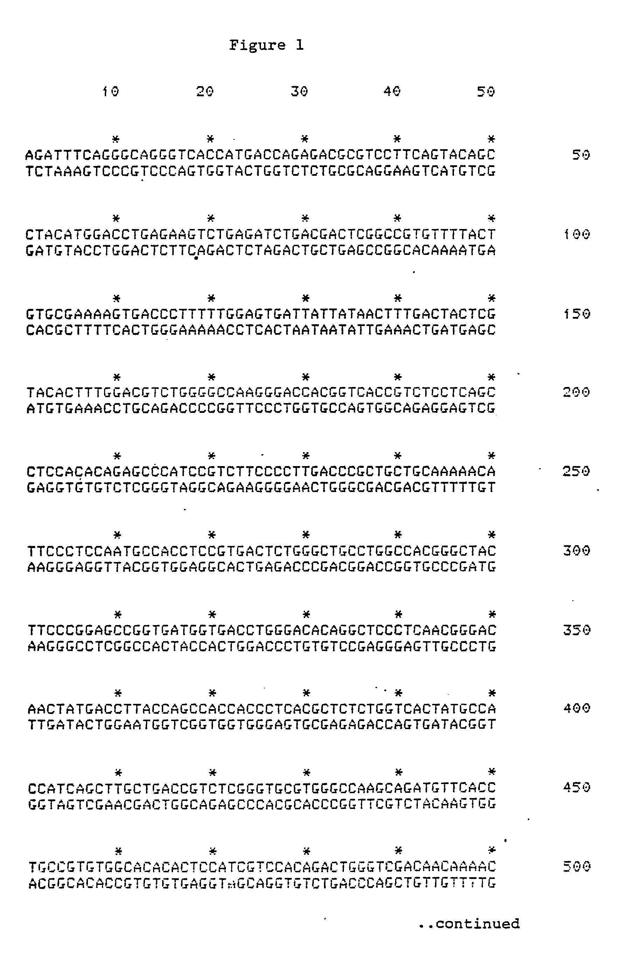

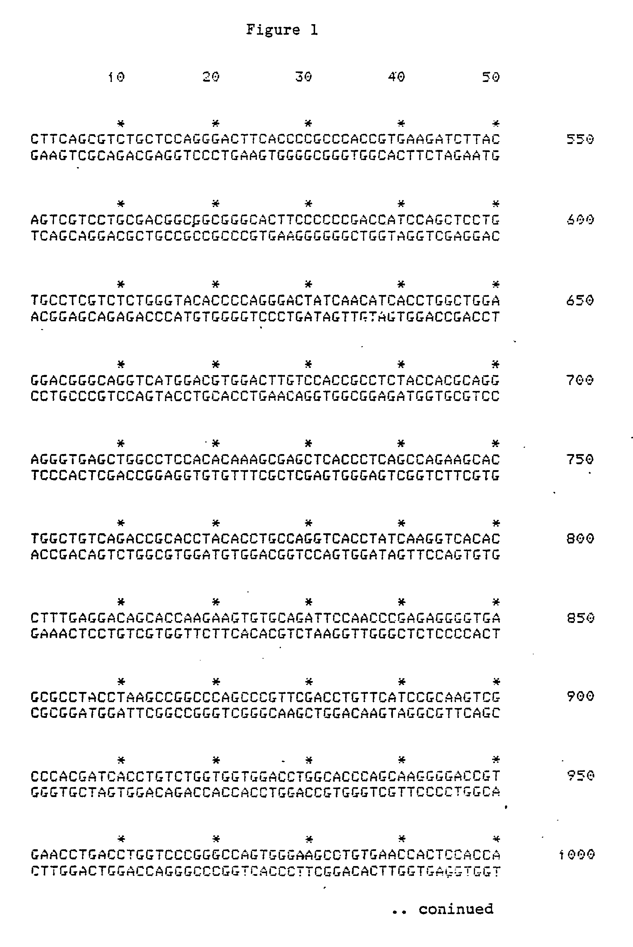

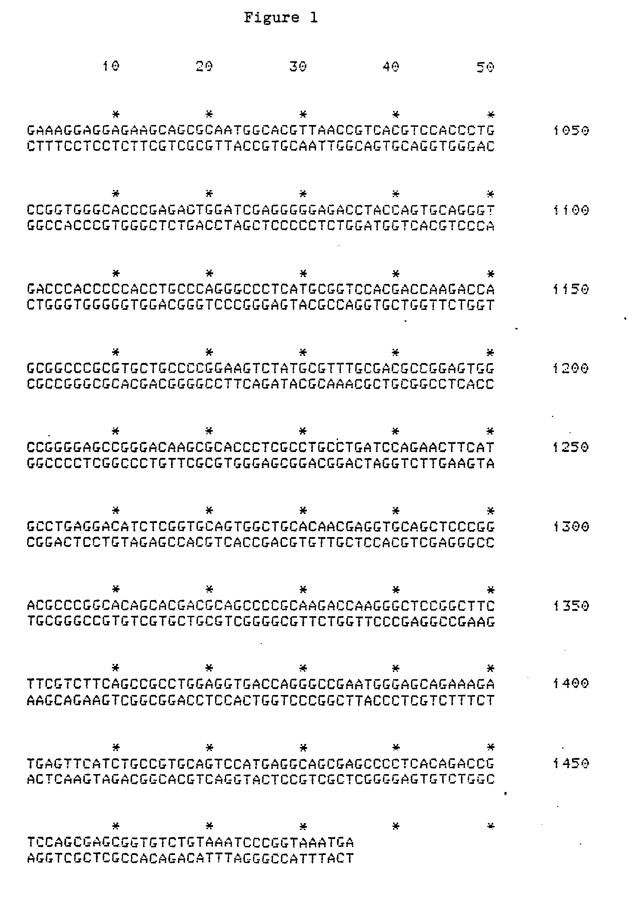

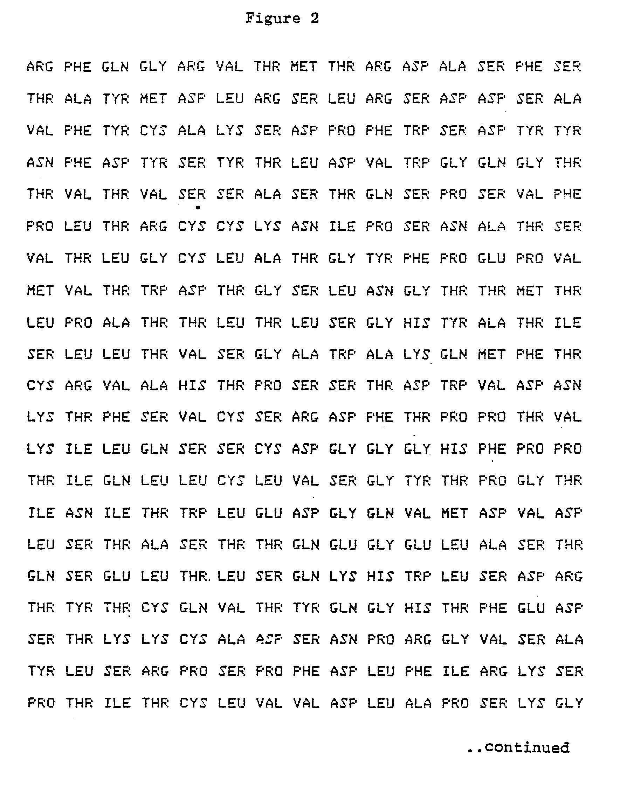

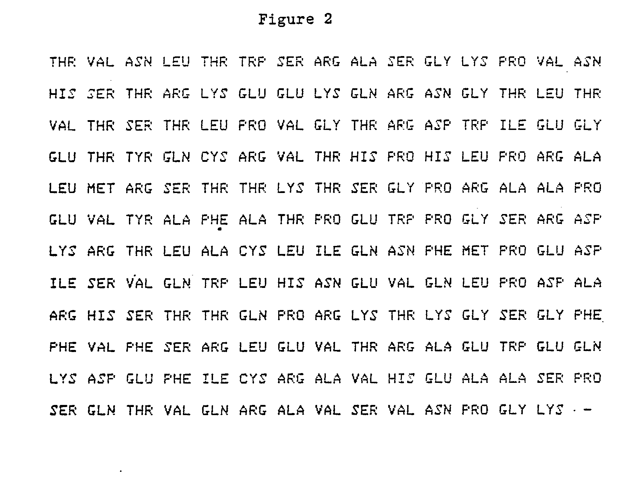

Referring to Fig. 1, the polynucleotide of the nucleotide sequence 831—1485 codes for a polypeptide of the amino acid sequence 278—494 as shown in Fig. 2. Thus, it codes for CH3—CH4 of the human IgE H-chain.

The polynucleotide of the nucleotide sequence 490—1485 as shown in Fig. 1 codes for a polypeptide of the amino acid sequence 164—494 as shown in Fig. 2, hence CH2—CH4 of the human IgE H-chain.

The polynucleotide of the nucleotide sequence 88—1485 as shown in Fig. 1 codes for a polypeptide of the amino acid sequence 30—494 as shown in Fig. 2. Said polypeptide covers the CH1—CH4 polypeptides of the human IgE H-chain.

Similarly, the polynucleotide of the nucleotide sequence 1—1485 as shown in Fig. 1 codes for a polypeptide of the amino acid sequence 1—494 as shown in Fig. 2. Said polypeptide includes the human IgE H-chain CH1—CH4 polypeptides.

For the direct expression, the above-mentioned polynucleotides may possess the codon ATG at the 5'-end thereof without reading frame shift. In that case, said polynucleotides code for polypeptides possessing Met at the N-terminus thereof.

The above-mentioned polynucleotides, with or without ATG at the 5'-end thereof without reading frame shift, are preferably linked at a site downstream from a promoter. The promoter includes, among others, the tryptophan synthesis (trp) promoter, rec A promoter and lactose promoter. Among these, the trp promoter is preferable.

Table 1 gives the definition of each symbol as used in the present specification, drawing and claims.

2

# 0 102 634

TABLE 1

| | | |
|---|---|---|
| DNA | = | deoxyribonucleic acid |
| cDNA | = | complementary deoxyribonucleic acid |
| RNA | = | ribonucleic acid |
| mRNA | = | messenger ribonucleic acid |
| A | = | (deoxy)adenylate |
| T | = | deoxythymidylate |
| G | = | (deoxy)guanylate |
| C | = | (deoxy)cytidylate |
| U | = | uridylate |
| dATP | = | deoxyadenosine triphosphate |
| dTTP | = | deoxythymidine triphosphate |
| dGTP | = | deoxyguanosine triphosphate |
| dCTP | = | deoxycytidine triphosphate |
| ATP | = | adenosine triphosphate |
| EDTA | = | ethylenediamine tetraacetate |
| SDS | = | sodium dodecyl sulfate |
| Gly | = | glycine |
| Ala | = | alanine |
| Val | = | valine |
| Leu | = | leucine |
| Ile | = | isoleucine |
| Ser | = | serine |
| Thr | = | threonine |
| 1/2 Cys | = | 1/2 cystine |
| Cys | = | cysteine |
| Met | = | methionine |
| Glu | = | glutamic acid |
| Asp | = | aspartic acid |
| Lys | = | lysine |
| Arg | = | arginine |
| His | = | histidine |
| Phe | = | phenylalanine |
| Tyr | = | tyrosine |
| Trp | = | tryptophan |
| Pro | = | proline |
| Asn | = | asparagine |
| Gln | = | glutamine |
| bp | = | base pair(s) |
| b | = | base(s) |

In the present invention, a double-stranded DNA coding for the human IgE H-chain polypeptide can be produced by synthesizing a single-stranded cDNA using the mRNA coding for the human IgE H-chain polypeptide as the template together with reverse transcriptase, for instance, then converting the cDNA to the double-stranded form, digesting the double-stranded DNA with an enzyme (exonuclease, endonuclease), adding an adapter to the digestion product, inserting the resulting product into a plasmid, introducing the plasmid into *Escherichia coli*, for instance, growing the thus-obtained transformant and isolating the cDNA-containing plasmid.

The mRNA to be used in the above process can be produced, for example, in the following manner.

Human myeloma cells of the established cell line U266, which are capable of producing human IgE, are cultivated, the proliferated cells are harvested by centrifugation, washed, for instance with physiological saline, and lysed in a denaturing solution, for instance N-lauroylsarcosine buffer, with heparin, diethyl pyrocarbonate, etc. added, and an RNA fraction is collected in the conventional manner by, for example, layering the lysate onto 5.7 M CsCl solution followed by centrifugation and extraction with phenol. Then, polyadenylic acid-containing RNAs are separated using oligo(dT)-cellulose, poly(U)-Sepharose or the like. The subsequent sucrose density gradient centrifugation gives the mRNA.

Using the thus-obtained mRNA as the template, a single-stranded cDNA is synthesized by any method known *per se* with the use of reverse transcriptase, and the cDNA is further converted to the double-stranded form [Maniatis, T. et al., Cell, *8*, 163 (1976)].

The double-stranded DNA is inserted into pBR 322 at the PstI or SphI restriction endonuclease cleavage site by, for example, the dG—dC or dA—dT homopolymer tailing method [Nelson, T. S., Methods in Enzymology, *68*, 41 (1979), Academic Press Inc., New York]. *Escherichia coli* strain χ1776, for instance, is transformed with the resulting recombinant plasmid. An adequate transformant can be selected on the basis of the tetracycline or ampicillin resistance.

3

The structural gene fragment for the human IgE H-chain has already been cloned [Nishida et al., Proc. Natl. Acad. Sci. USA, *79*, 3833 (1982)], and its nucleotide sequence has been partially analyzed. This gene fragment (gift from Prof. Tasuku Honjo of Osaka University, Faculty of Medicine) is labelled with $^{32}P$ by, for example, the nick translation method [Rigby, P. W. J. et al., J. Mol. Biol., *113*, 237 (1977)] or, alternatively, an oligo-nucleotide having the nucleotide sequence supposedly corresponding to the amino acid sequence of the human IgE H-chain polypeptide is synthesized chemically and labelled with $^{32}P$. With the labelled product as the probe, the desired clone is secondarily screened out from among the already obtained tetracycline- or ampicillin-resistant transformants by the *per se* known colony hybridization method [Grunstein, M. and Hogness, D. S., Proc. Natl. Acad. Sci. USA, *72*, 3961 (1975)]. The nucleotide sequence of the clone which gives a positive result in the above colony hybridization is determined by, for example, the method of Maxam-Gilbert [Maxam, A. M. & Gilbert, W., Proc. Natl. Acad. Sci. USA, *74*, 560 (1977)] or the dideoxy-nucleotide synthetic chain termination method using phage M13 [Messing, J. et al., Nucleic Acids Res., *9*, 309 (1981)], whereby the presence of the gene coding for the human IgE H-chain polypeptide can be confirmed. Then, the human IgE H-chain polypeptide-encoding gene can be cut out wholly or partly from the clone obtained and can be linked at a site downstream from an adequate promoter, the SD (Shine and Dalgarno) sequence and the translation start codon ATG, for introduction into an adequate host organism. The gene or part thereof can also be inserted into within an adequate structural gene (e.g. β-lactamase gene or anthranilate synthetase gene) as inserted in a plasmid. In that case, the expression product is a chimera polypeptide coupled with the whole or part of the structural gene product.

The promoter includes those mentioned hereinabove, and the host organism includes bacteria such as *Escherichia coli* and *Bacillus substilis*, among which *Escherichia coli* (e.g. strain 294, strain W3110), particularly strain 294, is preferred.

The strain 294 is a known strain [Backman, K. et al., Proc. Natl. Acad. Sci. USA, *73*, 4174 (1976)] and has been deposited with the Institute for Formentation, Osaka under deposit No. IFO-14171.

The transformation of a host organism with the DNA in the present invention is performed, for example, by the known method [Cohen, S. N. et al., Proc. Natl. Acad. Sci. USA. *69*, 2110 (1972)].

The thus-obtained transformant is cultivated in a *per se* known medium.

The medium is, for example, glucose- and Casamino acids-containing M9 medium [Miller, J., Experiments in Molecular Genetics, 431—433 (Cold Spring Harbor Laboratory, New York, 1972)]. An agent such as 3β-indolylacrylic acid may be added as necessary for increased promoter efficiency.

The cultivation is generally conducted at 15—43°C for 3 to 24 hours. Aeration and/or stirring may be made as necessary.

After cultivation, cells are harvested by the known method and, for instance after suspending in a buffer, destructed by, for example, treatment with lysozyme or a surface active agent or ultrasonic treatment, followed by centrifugation to give a supernatant.

The human IgE H-chain polypeptide can be isolated from said supernatant by any of the generally known methods of purifying proteins, more advantageously by anti-human IgE antibody column chromatography.

The human IgE H-chain polypeptide or a polypeptide equivalent thereto in immunological or biological activities as produced in the present invention is equivalent in immunological or biological activities to the human IgE H-chain polypeptide produced by the conventional method and can be used for the same purpose and in the same manner as the case where the conventional product is used.

Brief description of the drawing

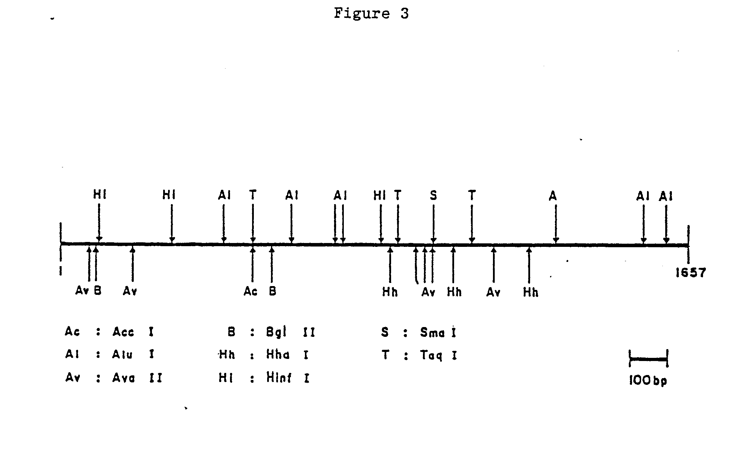

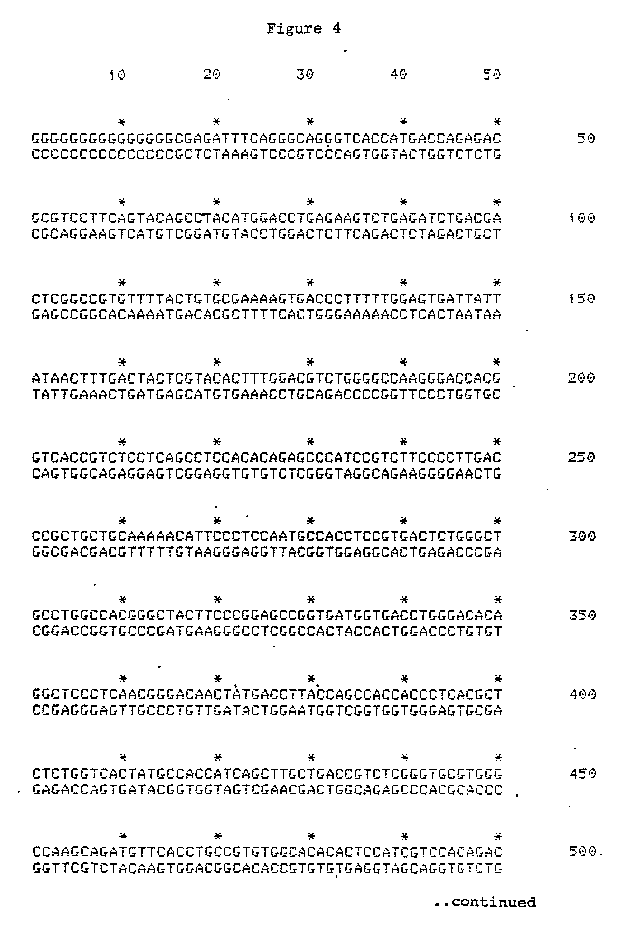

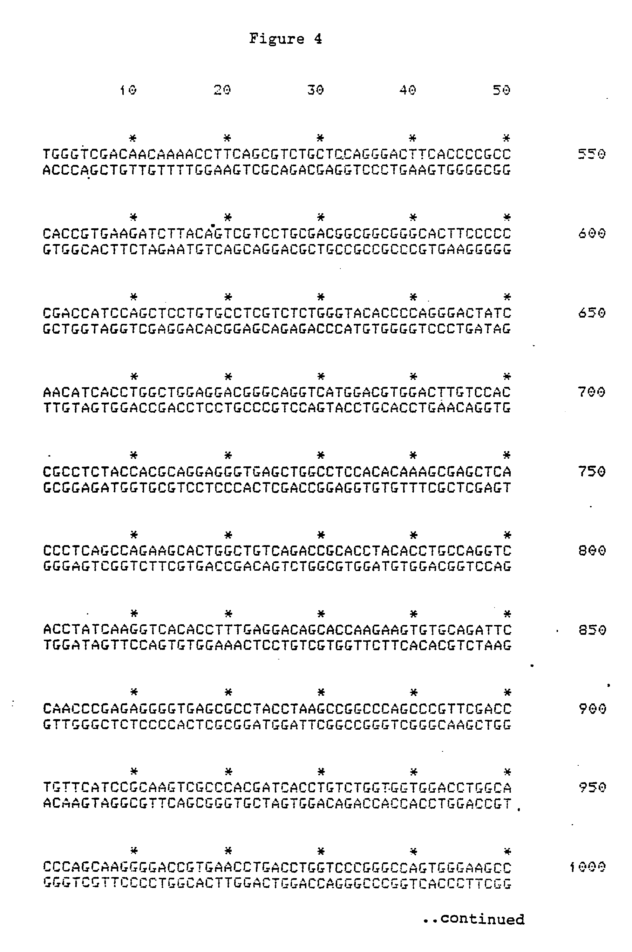

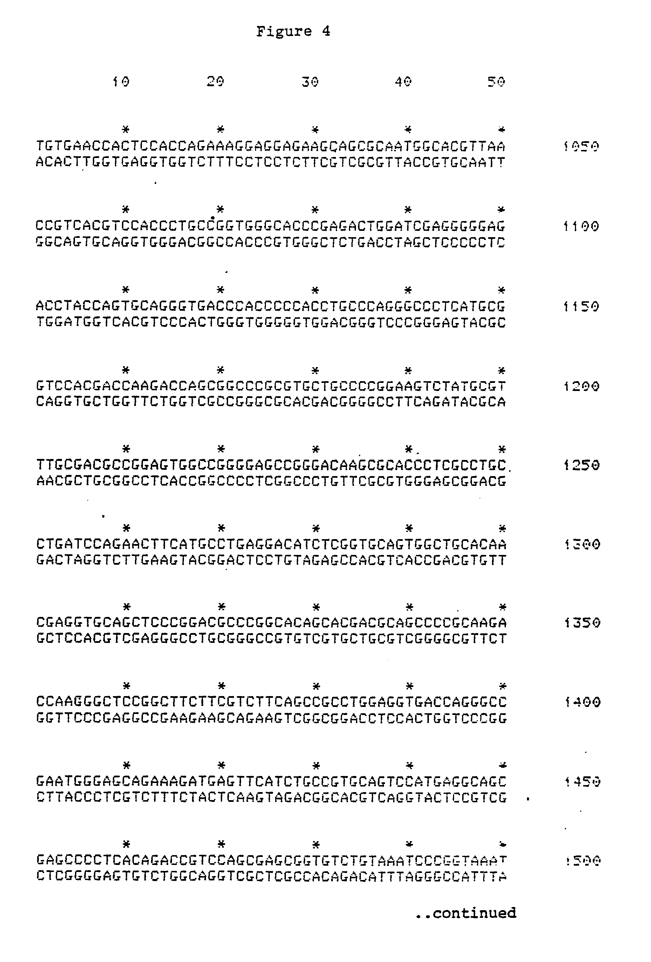

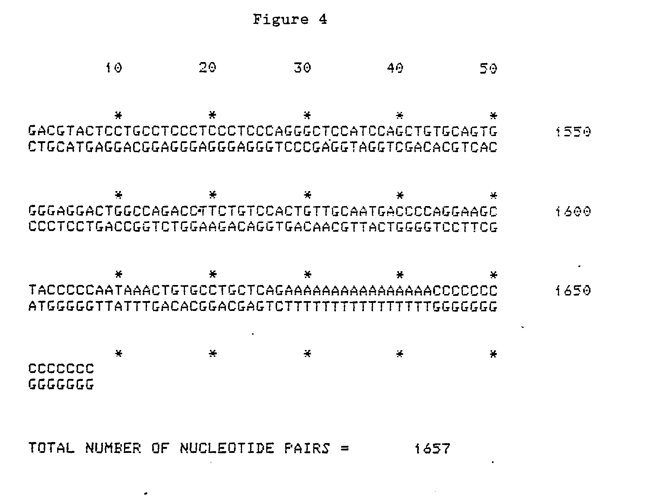

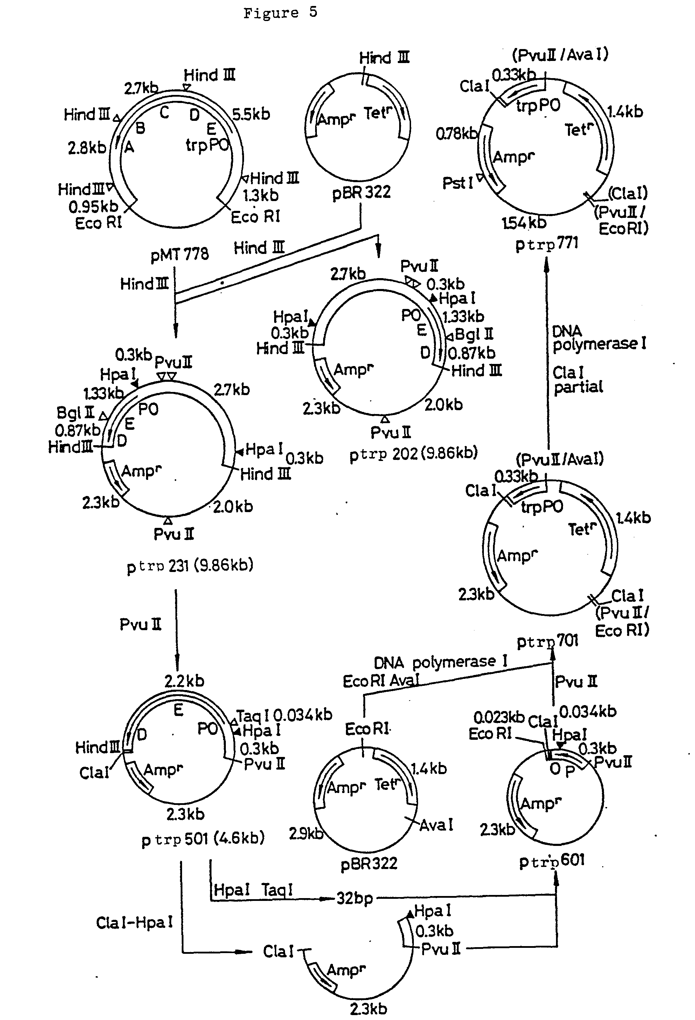

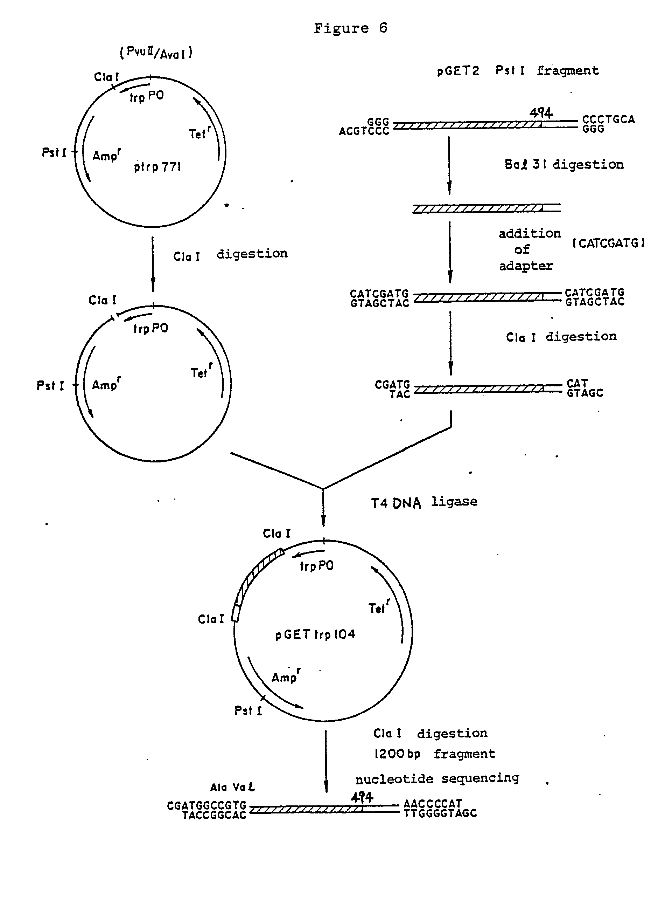

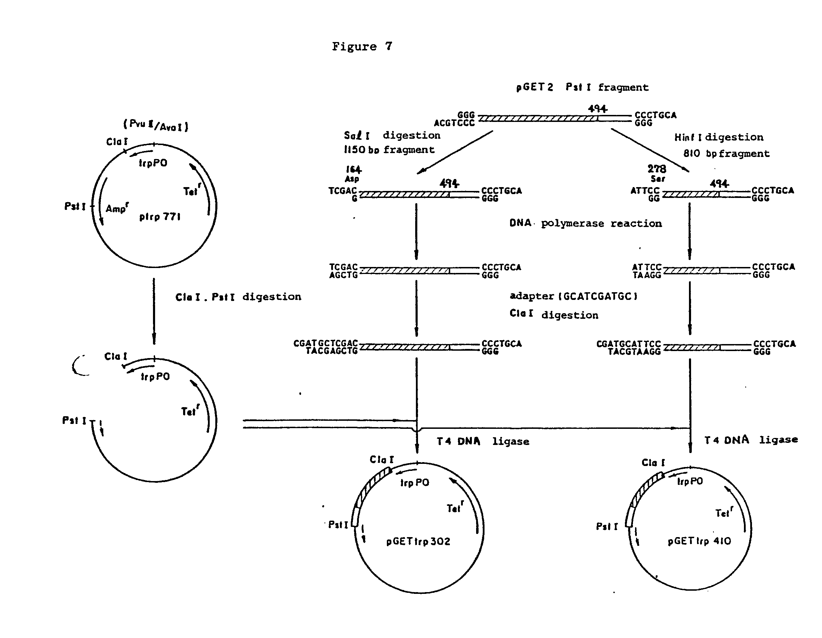

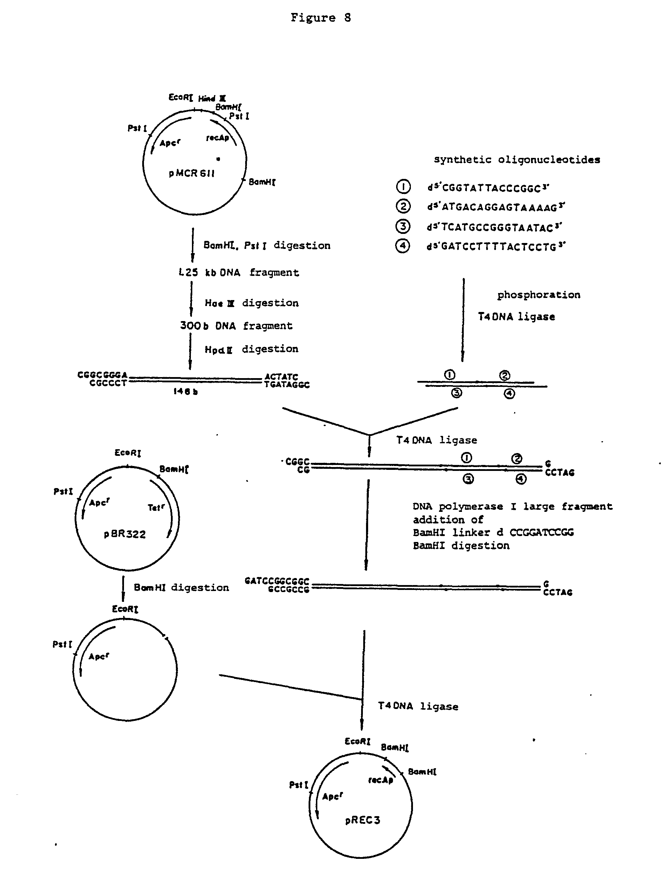

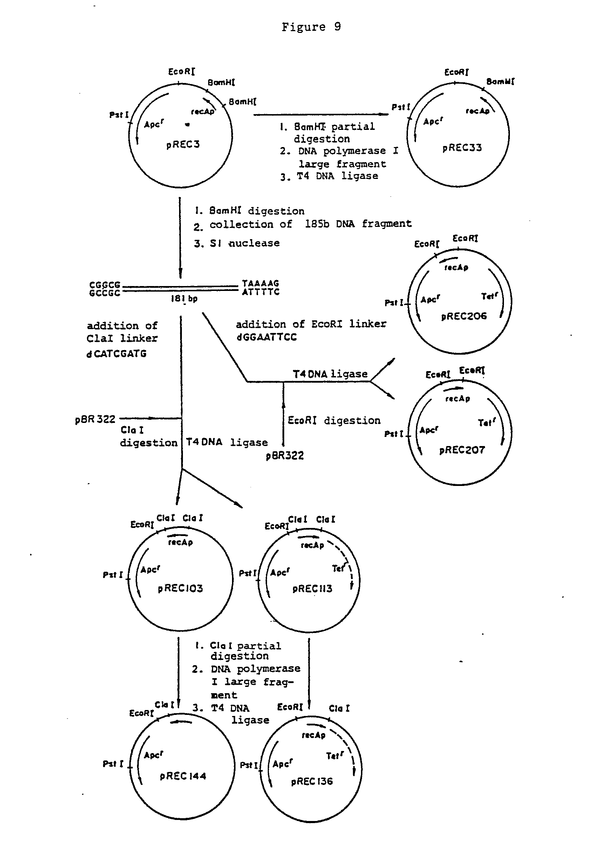

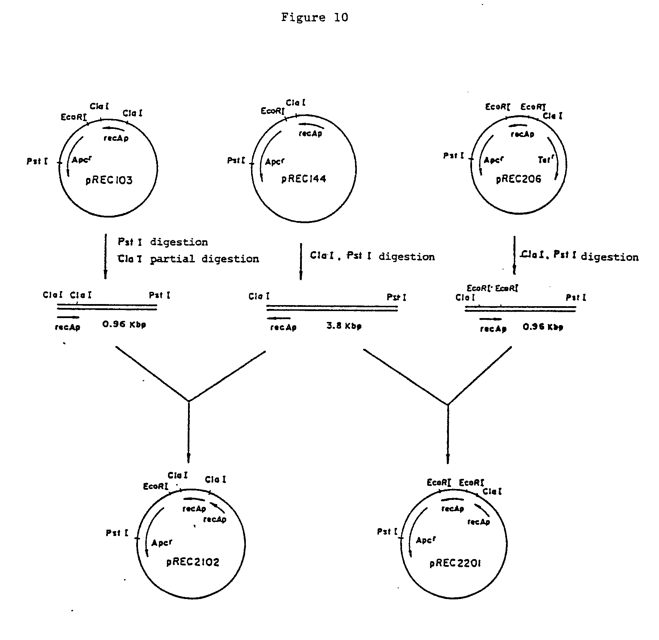

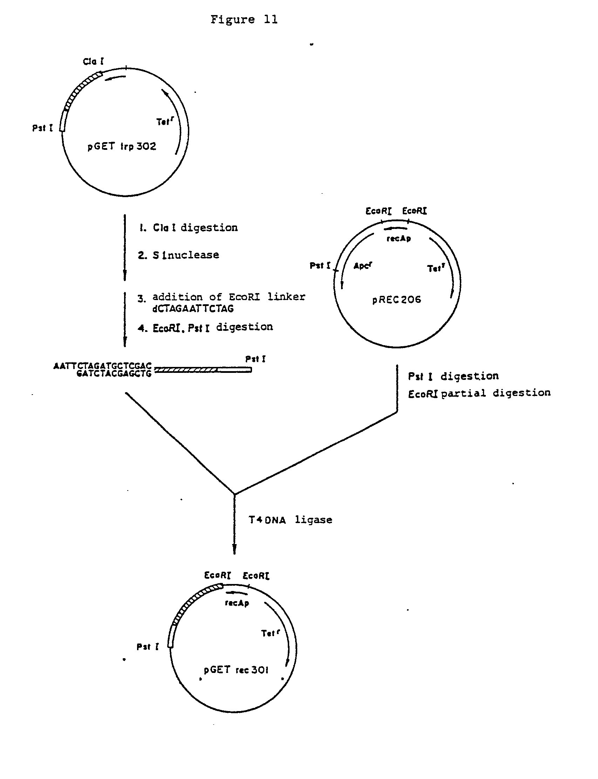

Fig. 1 illustrates the nucleotide sequence coding for the human IgE H-chain polypeptide, Fig. 2 illustrates the amino acid sequence corresponding to the nucleotide sequence shown in Fig. 1, Fig. 3 shows the restriction enzyme map for the cDNA in pGET2 as obtained in Example 1, and Fig. 4 illustrates the primary structure (nucleotide sequence) of said cDNA. Fig. 5 shows the construction scheme for the plasmid ptrp771, Fig. 6 shows the construction scheme in Example 2 and Fig. 7 shows the construction scheme in Example 3, the section indicated by ▨▨▨▨▨ , in each occurrence, being the fragment coding for the human IgE H chain polypeptide, Fig. 8 shows the construction scheme for pREC3, and Fig. 9 shows the construction scheme for pREC33, pREC206, pREC207, pREC103, pREC113, pREC114 and pREC136. Fig. 10 shows the construction scheme for the expression vectors pREC2102 and pREC2201 each containing two successive rec A promoters, Fig. 11 shows the construction scheme for a human immunoglobulin E H chain gene expression plasmid. The portion indicated by ▨▨▨▨▨ represents the IgE H chain-encoding DNA fragment.

Reference Example 1

Isolation of human IgE-enclosing mRNA

(1) Cultivation of U-266 cells

Human myeloma cells of the established cell line U-266 [Immunology, *38*, 63 (1979)] ($2.5 \times 10^5$ cells/ml) were cultivated in 500 ml of RPMI-1640 (Roswell Park Memorial Institute) medium with 10% fetal calf serum and 0.1 mg/ml each of penicillin and streptomycin (Takeda Chemical Industries) in a roller bottle at 37°C for 3 days.

**0 102 634**

(2) Preparation of polyadenylic acid-containing RNA

The total RNA extraction from U-266 cells was performed mainly by the method of Glisin et al. [Biochemistry, 13, 2633 (1974)]. Thus, U-266 cells after 3 days of were collected by centrifugation at 2,500 revolutions per minute for 5 minutes using a Sorvall centrifugal rotor GSA, suspended in physiological saline and again centrifuged again by the same manner for effecting cell washing. Five to ten volumes of 4% N-lauroylsarcosine buffer (Wako Pure Chemical Industries) [2 mg/ml heparin (Wako Pure Chemical Industries), 0.2% diethyl pyrocarbonate (Tokyo Kasei), 0.01 M Tris · HCl, pH 7.6] was added to the cells, and the cells were mashed 15—20 times using a 30-ml Teflon homogenizer. To the resulting solution was added CsCl to a concentration of 0.5 g/ml, and the solution was layered on 7 ml of 5.7 M CsCl in a centrifugal tube for use in a Spinco SW27 rotor and centrifuged at 26,000 revolutions per minute for 20 hours for RNA sedimentation. The supernatant in the tube was sucked off, the upper part of the tube was cut off so as to leave the lower part thereof (about 2 cm long), and the RNA sediment was dissolved in 0.4% N-lauroylsarcosine buffer. NaCl was added to the solution to a concentration of 0.2 M, and RNAs were precipitated at −20°C by adding cold ethanol to a final concentration of 70%.

(3) Fractionation by oligo(dT)-cellulose column chromatography

The ethanol-precipitated RNAs were collected by centrifugation on a Spinco SW27.1 rotor at 20,000 revolutions per minute for 20 minutes, and then dissolved in 10 ml of 10 mM Tris · HCl (pH 7.6)—0.5 M NaCl—1 mM EDTA—0.5% SDS buffer. A 10 ml syringe was packed with 4 ml (4 cm high) of oligo(dT)-cellulose dissolved in the same buffer. The above RNA solution was passed through this column and the eluent was again passed through the column to adsorb polyadenylic acid-containing RNAs. The column was washed with the same buffer until the ultraviolet absorption at 260 nm was no more detected, whereby unadsorbed RNAs were washed away. The polyadenylic acid-containing RNAs were then eluted from the column with 10 mM Tris · HCl (pH 7.6)—1 mM EDTA—0.3% SDS buffer (1 ml/fraction) while following the RNAs based on the absorption at 260 nm (O.D.). The RNA fractions were pooled and subjected to ethanol precipitation at −20°C.

(4) Fractionation by sucrose gradient centrifugation

About 2 mg of the polyadenylic acid-containing RNAs obtained by the above procedure was layered on 10—30% sucrose density gradient solution in 0.05 M NaCl—0.01 M EDTA—0.01 M Tris · HCl (pH 7.6)—0.2% SDS buffer, and centrifuged at 24,000 revolutions per minute and at 20°C for 22 hours using an SW27 rotor. Thereafter, the contents were divided into 40 fractions and, for each fraction, the absorption at 260 nm (O.D.) were measured. The fractions were pooled by fives with an about 18S fraction at the center and subjected to ethanol precipitation. In this manner, the desired mRNA was obtained.

Reference Example 2

Anti human IgE monoclonal antibody was prepared and allowed to bind to a water-insoluble carrier Affigel 10 (Bio-Rad) by the following procedure, namely, the procedure disclosed in Japanese Patent Unexamined Publication (Kokai) No. 96,028/1983 [Japanese Patent Application No. 193,324/1981 filed on November 30, 1981].

(1) Human myeloma cells of the established cell line U266 were inoculated into 40 roller bottles RC-94 [length 50 cm, diameter 11 cm; N.B.S. (USA)] each containing 500 ml of RPMI-1640 medium [Journal of American Medical Assocation, 199, 519(1967)] composed of 25 mM N-2-hydroxyethylpiperazine-N'-2-ethanesulfonic acid, 100 µg/ml kanamycin (Meiji) and 10% fetal calf serum to a concentration of $2.0 \times 10^5$—$2.5 \times 10^5$ cells/ml, and cultivated at 37°C for 4 to 5 days with revolving the bottles at 1 revolution per minute. When a concentration of the cells reached $4 \times 10^5$—$5 \times 10^5$ cells/ml, the culture was diluted about twice with the above medium to a concentration of $2 \times 10^5$—$2.5 \times 10^5$ cells/ml. Twenty liters of thus obtained cell suspension was cultivated under the same conditions as described above to subcultivate the cell line. The remaining suspension (20 l) was transferred to 8 l- and 15 l- Spinner flasks [Bellco Glass Inc. (USA)] and cultivated at 37°C for 5 to 6 days with revolving a stirrer in the flask at 75 revolutions per minute. The resulting culture fluid was subjected to continuous centrifugation (3000 rpm, flow rate 120 ml/min: Tomy contenuous centrifugate).

(2) The supernatant (40 l) as obtained in above two time-cultivations was subjected to salting out with ammonium sulfate (saturated with ammonium sulfate to a concentration of 40—55%) at 5°C. A precipitate was collected by centrifugation with a Beckmann model J-6B centrifugate (J.S-4.2 roter: 4000 rpm, 60 minutes). The precipitate was dissolved in 1,000 ml of phosphate buffered saline (PBS; 8.1 mM $NaH_2PO_4$, 1.5 mM $KH_2PO_4$, 2.7 mM KCl, 137 mM NaCl, pH 7.2), and then dialyzed against 10 l of the same buffer with one change of buffer at 5°C for 40 hours.

(3) A lysine-Sepharose 4B column (3.6×25.5 cm) prepared according to the method of Cuartrecasas et al. [Proc. Natl. Acad. Sci., USA, 61, 636 (1968)] was equilibrated with PBS containing 0.1% Tween 20. One sixth volume (200 ml) of the dialyzed solution in the dialyzer obtained in (2) was subjected to fractionation on the column. The elution of human IgE was performed with the same buffer (400 ml) and then the same buffer containing 20% dextrose (2,000 ml). The fractions containing the human IgE were pooled and concentrated with an ultrafilter membrane [Diaflo PM-10, Amicon Far East (USA)]. The concentrated solution (340 ml) was dialyzed against 4000 ml of 60 mM Tris-phosphate buffer (pH 8.0) with one change of buffer at 5°C for 40 hours.

(4) The dialyzed solution in the dialyzer (340 ml) obtained in (3) was diluted six times with distilled water, and then a half (1020 ml) of the diluted solution was subjected to fractionation on a DEAE-Sephacel column previously (4.8×28 cm) equilibrated with 10 mM Tris-phosphate buffer (pH 8.0). The column was washed with 1,000 ml of the same buffer, and the human IgE was eluted with a linear gradient prepared from 2,400 ml each of the same buffer and 70 mM Tris-phosphate buffer. The fractions (2,000 ml) containing the human IgE were quickly collected and ten times concentrated PBS (100 ml) was added. The mixture was concentrated to 30 ml with the ultrafilter membrane (Diaflo PM-10). The remaining diluted solution was treated with the same procedure to obtain 60 ml in total of the concentrated solution.

(5) The concentrated solution (60 ml) obtained in (4) was subjected to fractionation on a Sephacryl S-300 column (5×95 cm) previously equilibrated with PBS, and then the human IgE was eluted with the same buffer. The eluate containing the human IgE was concentrated to 40 ml with the ultrafilter membrane (Diaflo PM-10).

(6) The concentrated solution (40 ml) obtained in (5) was subjected to fractionation on the same Sephacryl S-300 column which was used in (5). After the eluate was circulated twice through the column as the absorption at 280 nm of the eluate from the column was monitored by an ultraviolet-ray absorption photometer [Uvicord S: LKB (Sweden)], the fractions containing the human IgE were collected with a fraction collector (recycling chromatography) and concentrated to 26 ml with the ultrafilter membrane [Diaflo PM-10].

(7) A Sepharose 4B column coupled with anti bovine serum rabbit IgG prepared according to the method of Cuartrecasas et al. was equilibrated with PBS, and the concentrated solution obtained in (6) was subjected to fractionation on the column. The fractions which passed through the column were collected and the column was washed with the same buffer (300 ml) containing 20% maltose. The fractions containing the human IgE were collected and concentrated to 15 ml with the ultrafilter membrane (Diaflo PM-10). This absorption procedure was repeated three times. This column was regenerated by washing with 5 M NaSCN (150 ml) and then PBS (200 ml).

(8) To reserve the purified human IgE stably for a long time, the concentrated solution obtained in (7) was distributed to vials (volume 2 ml) each containing 1 ml of the solution, followed by freeze-drying. The buffer used as a solvent contained 20% maltose.

(9) The purity of the human IgE obtained in (8) was not lower than 99% as judged by sodium dodecylsulfate-polyacrylamide gel electrophoresis [Laemmli, Nature, *227*, 680 (1970)]. Impurity proteins in this sample, such as bovine IgG or bovine IgA, derived from the medium were not more than 0.2% by immunochemical methods such as Ouchterlony [Progress in Allergy, *5*, 1 (1958)] or single radical immunoduffusion [Immunochemistry, *2*, 235 (1965)]. The yield of the human IgE from the supernatant of the culture was 29% (RIST method) [Immunology, *14*, 265 (1968)]. Physicochemical properties of the obtained human IgE were as follows.

i) Molecular weight 220,000±30,000

Molecular weight was measured by the above-mentioned polyacrylamide gel electrophoresis of Laemmli. As standard proteins for measurement or molecular weight, SDS-PAGE standard, High Molecular Standard (Bio-Rad, USA) was used. The human IgE was migrated as a single band or double according to the conditions of electrophoresis with a smaller relative mobility than that of myosin (M.W. 200,000).

ii) Ultraviolet absorption spectrum

The maximum of absorption at 280 nm and the minimum at 251 nm were observed.

iii) Isoelectric point around 7.4

The isoelectric point was measured by the chromato-focussing method.

iv) Composition of amino acids

Constant boiling HCl was added to the human IgE, and hydrolysis was allowed at 110°C for 24 hours. After hydrolysis, amino acid analysis was performed with a high-speed auto amino acid analyzer (Hitachi model 835). Cystine and cysteine were determined as cysteic acid after oxidation with performic acid followed by hydrolysis with HCl according to the method of Hirs [Methods in Enzymology, *11*, 197 (1967)]. The result is given in Table 2.

TABLE 2

| Amino acid | % mol |
|---|---|
| Lys | 4.3±0.2 |
| His | 2.3±0.3 |
| Arg | 4.5±0.2 |
| Asp | 7.6±0.1 |
| Thr | 11.0±0.4 |
| Ser | 11.9±0.7 |
| Glu | 9.4±0.5 |
| Pro | 6.8±0.5 |
| Gly | 7.0±0.5 |
| Ala | 7.3±0.2 |
| 1/2 Cys | 2.8±0.7 |
| Val | 6.9±0.3 |
| Met | 1.2±0.3 |
| Ile | 2.6±0.3 |
| Leu | 7.2±0.8 |
| Tyr | 3.4±0.4 |
| Phe | 3.4±0.2 |

v) Composition of sugars

As a result of measurement by the phenol-sulfate method [Analytical Chemistry, *28*, 350 (1956)], the content of neutral sugars was found to be 4.8±0.6% (calculated in terms of galactose). Individual quantitative analysis of neutral sugars and amino sugars was performed according to the method of Young and Hakomori [Journal of Biological Chemistry, *246*, 1192 (1971)] by gas chromatography (Hitachi model K-53]. Sialic acid was analyzed by the resorcinol periodate method [Journal of Biological Chemistry, *246*, 430 (1971)]. The result is given in Table 3.

TABLE 3

| Sugar | Weight % |
|---|---|
| fucose | 0.5±0.2 |
| mannose | 2.8±0.4 |
| galactose | 1.4±0.3 |
| N-acetylglucosamine | 4.1±1.0 |
| sialic acid | 1.0±0.8 |

(10) Female BALB/c mice aged 6—7 weeks were subcutaneously inoculated with 50 μg of the above-obtained human IgE in admixture with Freund's complete adjuvant. Two weeks after the inoculation, the mice were subcutaneously inoculated with 50 μg of the human IgE in admixture with Freund's

7

incomplete adjuvant. Further two weeks later, the human IgE (35 µg) was dissolved in a physiological saline and the mice were intraveneously inoculated with the solution. Three days later, spleen cells were collected in a conventional method. Thus obtained spleen cells ($1 \times 10^8$ cells) and $1 \times 10^7$ cells of myeloma P3-X63-Ag8-U1 resistant to 8-azaguanine derived from BALB/c mice in 0.3 ml of minimum essential medium (MEM) containing 45% polyethyleneglycol 6000 (PEG 6000) were left to stand and to react at 37°C for 7 minutes. Thereafter, MEM was added to the cell suspension as slowly as possible. After addition of 12 ml in total of MEM, the resulting mixture was centrifuged to collect the cells. The cells were suspended in RPMI-1640 medium containing 10% fetal calf serum maintained in advance at 37°C. Each of 48 wells on a multidish (Linbro) was seeded with 1 ml of the obtained suspension. Twenty-four hours later, a half of the culture supernatant was discarded from and 0.5 ml of fresh HAT medium (RPMI-1640 medium containing 10% fetal calf serum, $1 \times 10^{-4}$ M hypoxanthin, $4 \times 10^{-7}$ M aminopterine and $1.6 \times 10^{-5}$ M thymidine) was added to each well. Thereafter, a similar procedure was repeated every two or three days. Fourteen days after the multidish was seeded with the cells, the activity of anti human IgE antibody in each supernatant of well was measured. The cells from the wells which were positive in anti human IgE antibody activity were cloned by the limiting dilution method. Namely, cells were seeded on a multidish at rate of 0.2 cells per well. By the method, 31 hybridomas producing an anti human IgE antibody were obtained.

11) Female BALB/c mice aged 6 weeks was intraperitoneally inoculated with $1 \times 10^6$ cells of the obtained hybridomas (E235, 163 strain) producing anti human IgE antibody. Fourteen days later, ascites of the mice was collected. To the ascites (18 ml) obtained from 5 mice, ammonium sulfate was added to make a final concentration of 47%. The solution was centrifuged and the precipitate was collected. Thus obtained precipitate was dissolved in 6 ml of 20 mM Tris-HCl buffer (pH 7.8)—0.05 M sodium chloride. The solution was dialyzed against 2 l of the same buffer at 5°C for 16 hours. The resulting dialysate (15 ml) was subjected to fractionation on a DEAE-cellulose column ($1.6 \times 12$ cm) equilibrated in advance with the same buffer, elution being made with the same buffer, to give 65 mg of the anti human IgE monoclonal antibody. Thus obtained anti human IgE monoclonal antibody (15 mg) was allowed to bind to a water-insoluble carrier Affigel 10.

Example 1
(i) Synthesis of single-stranded DNA
A mixture of 5 µg of the mRNA as obtained in Reference example 1, 100 units of reverse transcriptase (Life Science) and 100 µl of reaction mixture [5 µg of oligo(dT), 1 mM each of dATP, dCTP, dGTP and dTTP, 8 mM $MgCl_2$, 50 mM KCl, 10 mM dithiothreitol, 50 mM Tris · HCl, pH 8.3] was incubated at 42°C for an hour, then deproteinized with phenol, and treated with 0.1 N NaOH at 70°C for 20 minutes for decomposing and removing the RNA.

(ii) Synthesis of double-stranded DNA
The thus-synthesized single-stranded complementary DNA was maintained in 50 µl of a reaction mixture [the same reaction mixture as above except for the absence of the mRNA and oligo(dT)] at 42°C for 2 hours, whereby a double-stranded DNA was synthesized.

(iii) Addition of dC tail
This double-stranded DNA was subjected to the reaction of 60 units of nuclease S1 (Bethesda Research Laboratories) in 50 µl of a reaction mixture (0.1 M sodium acetate, pH 4.5, 0.25 M NaCl, 1.5 mM $ZnSO_4$) at room temperature for 30 minutes. The reaction mixture was then deproteinized with phenol, and the DNA was precipitated with ethanol. The DNA was subjected to the reaction of 30 units of terminal transferase (Bethesda Research Laboratories) in a reaction mixture [0.14 M potassium cacodylate, 0.3 M Tris (base), pH 7.6, 2 mM dithiothreitol, 1 mM $CoCl_2$, 0.15 mM dCTP] at 37°C for 3 minutes, whereby about 20 deoxycytidylates were linked to each 3'-end of the double-stranded DNA. The above series of reactions gave about 300 ng of a deoxycytidylate chain-bearing double-stranded DNA.

(iv) Cleavage of *Escherichia coli* plasmid and addition of dG tail
Separately, 10 µg of *Escherichia coli* plasmid pBR322 DNA was subjected to the reaction of 20 units of the restriction enzyme PstI in 50 µl of a reaction mixture [50 mM NaCl, 6 mM Tris · HCl (pH 7.4), 6 mM $MgCl_2$, 6 mM 2-mercaptoethanol, 100 µg/ml bovine serum albumin] at 37°C for 3 hours, whereby the pBR322 DNA was cleaved at the PstI recognition site. After deproteinization with phenol, the cleavage product was further subjected to the reaction of 30 units of terminal transferase in 50 µl of a reaction mixture [0.14 M potassium cacodylate, 0.3 M Tris (base), pH 7.6, 2 mM dithiothreitol, 1 mM $CoCl_2$, 0.15 mM dGTP] at 37°C for 3 minutes, whereby the above plasmid pBR322 DNA was extended by about 8 deoxyguanylates at each 3'-end.

(v) Annealing of cDNA and *Escherichia coli* plasmid and transformation of *Escherichia coli*
The thus-obtained synthetic double-stranded DNA (0.1 µg) and the above plasmid pBR322 DNA (0.5 µg) were annealed together by heating in a solution comprising 0.1 M NaCl, 50 mM Tris · HCl, pH 7.6, and 1 mM EDTA at 65°C for 2 minutes and then at 45°C for 2 hours, followed by slow cooling. The transformation

of *Escherichia coli* χ1776 was performed according to the method of Enea et al. [J. Mol. Biol., *96*, 495 (1975)].

(vi) Isolation of cDNA-containing plasmid

In this way, 1445 tetrcycline-resistant colonies were isolated. The DNA of each of them was fixed on a nitrocellulose filter (vide supra).

Separately, the gene fragment corresponding to the human IgE H-chain polypeptide (vide supra) was labelled with $^{32}$P by the nick translation method (vide supra).

The DNA (0.2 µg) was treated in 25 µl of a reaction mixture [50 mM Tris-HCl, pH 7.5, 5 mM MgCl$_2$, 1 mM 2-mercaptoethanol, 1.0 µCi α-$^{32}$P-dATP, 0.4 ng bovine pancreatic DNase I (Worthington)] at room temperature for 2 minutes. Then, 25 units of *Escherichia coli* DNA polymerase I (Boehringer Mannheim) was added and the reaction was conducted at 15°C for 30 minutes. Purification by extraction with phenol and precipitation with ethanol gave a uniformly $^{32}$P-labelled DNA.

With this $^{32}$P-DNA as the probe, this was annealed with the DNA fixed on the nitrocellulose filter according to the method of Lawn et al. [Nucleic Acids Res., *9*, 6103 (1981)]. As a result of autoradiography, 9 colonies responding to the probe were isolated and named pGET 1 to 9, respectively.

The plasmid DNA was isolated from cells of each of these colonies by the method of Birnboim-Doly [Birnboim, H. C. and Doly, J., Nucleic Acids Res. *7*, 1513 (1979)]. Then, the insert was cut out from the plasmid DNA using the restriction enzyme PstI, whereby, among the plasmids separated, pGET2 DNA was found to contain the longest insert. Accordingly, the pGET2 DNA was selected for further use.

The restriction enzyme cleavage map of the cDNA inserted in this plasmid is as shown in Fig. 3. The primary structure (nucleotide sequence) of the cDNA sequence as inserted in the pGET2 plasmid was determined by the dideoxynucleotide synthetic chain termination method and by the method of Maxam-Gilbert. The nucleotide sequence thus determined is as shown in Fig. 4. The polynucleotide of the nucleotide sequence 18—1502 as shown in Fig. 4 corresponds to the polynucleotide as shown in Fig. 1.

The amino acid sequence which this nucleotide sequence codes for, when there is no reading frame shift, is approixmately equal to the amino acid sequence of the IgE H-chain polypeptide as reported by Dorrington et al. [Immunological Rev., *41*, 3 (1978)]. This confirms that the cDNA inserted in pGET2 codes for the IgE H-chain polypeptide. This cDNA begins with the codon coding for the 63th amino acid in the V region of the IgE H-chain as reported by Dorrington et al. (vide supra), hence wholly codes for the C region. Furthermore, it is believed that it retains the whole structure on the 3'-end side of the mRNA, inclusive of non-coding regions, since the poly(A) structure is present.

Therefore, the C-region polypeptide which carries the antigenicity of human IgE can be produced by adding the translation start condon ATG to the 5'-end of the nucleotide sequence inserted in the above plasmid, without reading frame shift, followed by insertion into another expression plasmid and transformation of *Escherichia coli*, for instance, therewith.

Example 2

The insert in the plasmid pGET2 as obtained in Example 1 was cut out using the restriction enzyme PstI. This DNA fragment (2 µg) was partially digested from both ends under the reaction of 2 units of nuclease Bal 31 [New England Biolabs; Gray et al., Nucleic Acids Res., *2*, 1459 (1975)] in 60 µl of a reaction mixture (20 mM Tris · HCl, pH 8.0, 0.6 M NaCl, 12 mM CaCl$_2$, 12 mM MgCl$_2$, 1 mM EDTA) at 30°C for 1 minute.

The DNA was extracted from the reaction mixture with phenol and purified by precipitation with ethanol, and then joined with the adapter $^5$'CATCGATG$^3$', which contains the translation start codon and restriction enzyme ClaI-recognition site, using T4 DNA ligase (New England Biolabs).

Separately, the plasmid ptrp771 as an expression plasmid (the vector being pBR322), which contains an *Escherichia coli* trp promoter portion [promoter- and operator-containing 276 bp DNA fragment; Bennett, G. N. et al., J. Mol. Biol., *121*, 113 (1978)], was constructed by the following method, namely, the method disclosed in European Patent Publication No. 68,719 [Japanese Patent Application No. 85,280/1982 filed on May 19, 1982]

(1) 2 µg of plasmid pMT778 DNA with the tryptophan operon inserted therein was cleaved with restriction enzyme HindIII (Takara Shuzo) at 37°C for 2 hours in 20 µl of a reaction mixture (10 mM Tris-HCl, pH 7.5, 7 mM MgCl$_2$, 60 mM NaCl, 2 units HindIII) and then electrophoresed using 0.7% agarose slab gel. After the electrophoresis, a 5.5 kbp DNA fragment containing trp promoter, operator, trp E and part of trp D was recovered from the gel.

1 µg of plasmid pBR322 DNA was converted to a linear DNA form by digestion with restriction enzyme HindIII in 20 µl of a reaction mixture (10 mM Tris-HCl, pH 7.5, 7 mM MgCl$_2$, 60 mM NaCl, 2 units HindIII) at 37°C for two hours, and then treated with 0.1 unit of alkaline phosphatase (Worthington, U.S.A) at 65°C for 30 minutes, whereby the 5'-terminal phosphate residue was removed. The reaction mixture was deproteinized with phenol, and the DNA (HindIII-digested pBR322) was precipitated with cold ethanol.

The HindIII-digested pBR322 DNA (400 ng) and the above 5.5 kbp DNA fragment (200 ng) were mixed and treated for DNA ligation in 10 µl of a reaction mixture [66 mM Tris-HCl, pH 7.6, 6.6 mM MgCl$_2$, 10 mM dithiothreitol, 1 mM ATP, 2 units T4 DNA ligase (Takara Shuzo)] at 14°C overnight. The reaction mixture was used for transformation of *Escherichia coli* 294 by the above-cited method of Cohen et al. Among the transformants selected on the basis of amplicillin resistance as an index, there were found a strain resistant only to amplicillin and a strain resistant to both ampicillin and tetracycline. Plasmid DNA from the cells of each strain by the method of Birnboim and Doly (vide supra) was isolated and the molecular weight and restriction enzyme cleavage pattern were investigated in detail. Plasmid ptrp231 DNA with the 5.5 kbp DNA fragment inserted therein in the same direction as the β-lactamase gene and plasmid ptrp202 with said DNA fragment inserted therein in the same direction as the tetracycline resistance gene were detected in the former strain and in the latter one, respectively (cf Fig. 5).

(2) 1 µg of plasmid ptrp231 DNA was cleaved with restriction enzyme Pvull (Takara Shuzo) in 20 µl of a reaction mixture (10 mM Tris-HCl, pH 7.5, 7 mM MgCl$_2$, 60 mM NaCl, 7 mM 2-mercaptoethanol, 2 units Pvull) at 37°C for 2 hours. The reaction mixture was deproteinized with phenol, and DNA was precipitated with cold ethanol. This DNA was religated with T4 DNA ligase under the conditions described in above (1). The reaction mixture was used for transformation of Escherichia coli 294. Among ampicillin-resistant transformants, there was obtained plasmid ptrp501 in which a ca. 5.1 kbp Pvull DNA fragment of plasmid ptrp231 was removed (cf Fig. 5).

(3) 5 µg of plasmid ptrp501 DNA was cleaved with restriction enzymes Clal (Boehringer Mannheim, West Germany) and Hpal in 50 µl of a reaction mixture (10 mM Tris-HCl, pH 7.5, 7 mM MgCl$_2$, 100 mM KCl, 7 mM 2-mercaptoethanol, 0.01% bovine serum albumin, 5 units Clal, 5 units Hpal) at 37°C for 2 hours. Then, 0.5 unit of alkaline phosphatase was added and the reaction was carried out at 65°C for 30 minutes. The ·reaction mixture was subjected to 0.7% agarose slab gel electrophoresis and a 2.6 kbp DNA fragment was recovered from the gel. Separately, 50 µg of plasmid ptrp501 DNA was cleaved with restriction enzyme Hpal in 200 µl of a reaction mixture (10 mM Tris-HCl, pH 7.5, 7 mM MgCl$_2$, 100 mM KCl, 7 mM 2-mercaptoethanol, 5 units Hpal) at 37°C overnight. Then, 50 units of restriction enzyme Taql was added and the reaction was continued at 65°C for 2 hours. Using 10% polyacrylamide slab gel, the reaction mixture was electrophoresed in a buffer (89 mM Tris-HCl, 89 mM boric acid, 2.5 mM EDTA, pH 8.3) at 160 V for 1.5 hours. After the electrophoresis, a 32 bp DNA fragment was recovered from the gel.

200 ng of the 2.6 kbp Clal-Hpal DNA fragment and 1.5 ng of the Clal-Taql DNA fragment were mixed and subjected to DNA ligation with T4 DNA ligase under the conditions described in above (1). Using the reaction mixture, Escherichia coli 294 was transformed, and plasmid ptrp601 DNA was obtained from the ampicillin-resistant transformant (cf Fig. 5).

(4) 1 µg of plasmid ptrp601 DNA was cleaved with restriction enzyme Pvull in 20 µl of a reaction mixture (10 mM Tris-HCl, pH 7.5, 7 mM MgCl$_2$, 60 mM NaCl, 7 mM 2-mercaptoethanol, 2 units Pvull) at 37°C for 2 hours. The reaction mixture was deproteinized with phenol and the DNA was precipitated with cold ethanol. Separately, 2 µg of plasmid pBR322 DNA was cleaved with restriction enzymes EcoRI (Takara Shuzo) and Aval (New England BioLabs) in 20 µl of a reaction mixture (100 mM Tris-HCl, pH 7.5, 7 mM MgCl$_2$, 50 mM NaCl, 7 mM 2-mercaptoethanol, 2 units EcoRI, 2 units Aval) at 37°C for 2 hours. The reaction mixture was subjected to 0.7% agarose slab gel electrophoresis. After the electrophoresis, a 1.4 kbp EcoRI-Aval DNA fragment was recovered from the gel.

500 ng of the 1.4 kbp EcoRI-Aval DNA fragment was filled in with DNA polymerase large fragment (New England BioLabs) in 30 µl of a reaction mixture (40 mM potassium phosphate buffer, pH 7.5, 6.6 mM MgCl$_2$, 1 mM 2-mercaptoethanol, 33 µM dATP, 33 µM dGTP, 33 µM dTTP, 33 µM dCTP, 2 units Klenow's DNA polymerase I) at room temperature for 30 minutes, whereby the cohesive ends were rendered blunt. 200 ng of said 1.4 kbp DNA fragment and 200 ng of Pvull-digested ptrp601 DNA were mixed and subjected to ligation with T4 DNA ligase under the conditions described in above (1). Escherichia coli 294 was transformed with the reaction mixture, and plasmid ptrp701 DNA was obtained from the ampicillin- and tetracycline-resistant transformant (cf Fig. 5).

(5) 2 µg of plasmid ptrp701 DNA was partially digested with diluted restriction enzyme Clal in 20 µl of a reaction mixture (10 mM Tris-HCl, pH 8.0, 10 mM MgCl$_2$, diluted Clal) at 37°C for 15 minutes. Following deproteinization with phenol, the DNA was precipitated with ethanol. The cohesive ends of this partially cleaved ptrp701 DNA was filled in with Klenow's DNA polymerase I under the conditions described in above (4). Following deproteinization with phenol, the DNA was precipitated with cold ethanol. The DNA was religated with T4 DNA ligase under the conditions described in above (1). Escherichia coli 294 was transformed with the reaction mixture, and plasmid ptrp771 having only one Clal site as compared with two Clal sites originally present in plasmid ptrp701 DNA was obtained from the thus-obtained transformant.

This expression plasmid ptrp771 was cleaved with the restriction enzyme Clal. Thereinto, at the cleavage site, inserted was the above-mentioned pGET2 insert DNA-adapter joining product, which also had been cleaved with Clal, with the use of T4 DNA ligase (Fig. 6). Using the reaction product, Escherichia coli 294 was transformed according to the method of Cohen et al. (vide supra). There were obtained a large number of colonies containing plasmids differing in the nuclease Bal 31 digestion region.

Human IgE H-chain polypeptide-producing colonies were selected from among the thus-obtained colonies by the colony immunoassay method [Kemp, D. J., and Cowman, A. F., Proc. Natl. Acad. Sci. USA, 78, 4520 (1981)]. Thus, the colonies grown on a nitrocellulose filter were lysed by contacting with a 0.1 M NaHCO$_3$-0.1% Triton X100-lysozyme (200 µg/ml) solution and directly transferred onto a cyanogen bromide-activated filter paper (Whatman No. 540) for fixation of the colonies on the filter paper. The filter paper was reacted with goat antihuman IgE antibody (Miles), then washed with a washing solution (50 mM

Tris · HCl, pH 8.0, 0.5 M NaCl, 0.1% Triton X100, 1% bovine serum albumin), and further reacted with [125]I-labelled protein A (RCC Amersham, Great Britain). After the reaction, the filter paper was washed well and autoradiographed.

The plasmid contained in the colony that reacted most positively with the anti-human IgE antibody in the above assay was named pGETtrp104. This plasmid was extracted from cells by the method of Birnboim-Doly (vide supra). The nucleotide sequence coding for the human IgE H-chain, which was inserted in ptrp771 and now existing in this pGETtrp104, was determined by the dideoxynucleotide synthetic chain termination method (vide supra). It was revealed that the human IgE H-chain polypeptide-encoding polynucleotide starting with the codon for the 92nd amino acid (according to the report by Dorrington) is located following the translation start codon without reading frame shift and that the poly(A) structure at the end of the mRNA structure is retained on the 3'-end side (Fig. 4). *Escherichia coli* 294/pGETtrp104 has been deposited with the Institute for Fermentation, Osaka under deposit No. IFO 14284.

Example 3

(i) From the plasmid pGET2 as obtained in Example 1, the insert was cut out with the restriction enzyme PstI. This DNA fragment was further cleaved with the restriction enzyme SalI. There was thus obtained an about 1.150 bp DNA fragment having the SalI site at one end and the PstI site at the other. The single-stranded cohesive DNA terminus at the SalI site of this DNA fragment was filled in with *Escherichia coli* DNA polymerase I large fragment and the DNA fragment was joined with the adapter 5'GCATCGATGC3' containing the translation start codon and restriction enzyme ClaI recognition site with the use of T4 DNA ligase (New England Biolabs). The joining product was cleaved with the restriction enzyme ClaI and then joined with the expression plasmid ptrp771 cleaved in advance with the restriction enzyme ClaI and PstI, with the use of T4 DNA ligase (Fig. 7). The above series of reactions resulted in construction of a human IgE H-chain polypeptide expression plasmid, pGETtrp302, containing the translation start codon and the Leu-encoding codon CTC newly introduced without reading frame shift at a site downstream from the trp promoter, and coding for the human IgE H-chain polypeptide starting from the 218th amino acid according to the report of Dorrington. *Escherichia coli* 294 was transformed with this expression plasmid according to the method of Cohen et al. to give a desired strain carrying the plasmid pGETtrp302. *Escherichia coli* 294/pGETtrp302 has been deposited with the Institute for Fermentation, Osaka under deposit No. IFO-14285.

(ii) From the plasmid pGET2 as obtained in Example 1, the insert was cut out with the restriction enzyme PstI. This DNA fragment was further cleaved with the restriction enzyme HinfI. There was thus obtained an about 810 bp DNA fragment with the HinfI site at one end and the PstI site at the other.

The single-stranded cohesive DNA terminus at the HinfI site of this DNA fragment was filled in with *Escherichia coli* DNA polymerase I large fragment (Bethesda Research Laboratories) so as to render the end blunt, and then the DNA fragment was joined with the same adapter as used in Example 3-(I), i.e. 5'GCATCGATGC3', with the use of T4 DNA ligase.

The joining product was cleaved with the restriction enzyme ClaI and joined with the expression plasmid ptrp771 cleaved in advance with the restriction enzymes ClaI and PstI, with the use of T4 DNA ligase (Fig. 7). The above series of reactions resulted in construction of a human IgE H-chain polypeptide expression plasmid, pGETtrp410, containing the translation start codon and the codon CAT for His at a site downstream from the trp promoter, and coding for the human IgE H-chain polypeptide starting with the 331st amino acid according to Dorrington without reading frame shift. *Escherichia coli* 294 was transformed with this plasmid according to the method of Cohen et al. to give a desired strain carrying the plasmid pGETtrp410. *Escherichia coli* 294/pGETtrp410 has been deposited with the Institute for Fermentation, Osaka under deposit No. IFO-14286.

Example 4

The IgE H-chain expression plasmid-carrying strains as obtained in Examples 2 and 3 were cultivated in 20 ml of M9 medium containing 1% glucose and 0.4% Casamino acids at 37°C for 4 hours. Then, indolyl acrylic acid was added to a concentration of 30 μg/ml, and the cultivation was continued at 37°C for 3 hours. Cells were harvested, washed with saline, and lysed by suspending in 0.5 ml lysing solution (10 mM Tris · HCl, pH 8.0, 10 mM EDTA, 0.2 M NaCl, 1 mM phenylmethylsulfonyl fluoride, 0.02% Triton X100, 0.1 mg/ml lysozyme) and allowing the suspension to stand at 0°C for 45 minutes and then at 37°C for 2 minutes. The lysate was further subjected to slight (30-second) ultrasonic treatment for breaking celluler DNAs which were dissolved. The lysate was then centrifuged at 4°C at 15,000 rpm (Servall SS34 rotor) for 30 minutes. The thus-obtained supernatant was assayed for IgE activity by the RIST method (vide supra) using an IgE assay kit (IgE Test-Shionogi; Shionogi).

The results are shown in Table 4. The strain carrying pGETtrp302 produced the human IgE H-chain polypeptide at the highest rate (480 ng/ml extract).

TABLE 4

| Transformant | IgE H-chain production (ng/ml extract) |
|---|---|
| E. coli 294/ptrp771 | 0 |
| E. coli 294/pGETtrp104 | 84 |
| E. coli 294/pGETtrp302 | 480 |
| E. coli 294/pGETtrp410 | 48 |

Example 5

5 ml of the extract from pGETtrp302-carrying cells as obtained in Example 4 was treated on a 1-ml column of the anti-human IgE monoclonal antibody-Affigel 10 prepared in Reference Example 2. The column was washed with 50 ml of PBS (20 mM phosphate buffer, pH 6.8, 0.15 M NaCl) containing 20% dextrose, and the human IgE H-chain adsorbed on the column was eluted from the column with 5 ml of 0.2 M acetic acid-0.15 M NaCl solution. The eluate was immediately neutralized, and dialyzed against 1 liter of PBS at 5°C for 24 hours. This procedure gave the human IgE H-chain polypeptide in a purity of not lower than 80% at a recovery rate of about 50%.

Example 6

(1) Preparation of rec A promoter-containing DNA fragment (cf. Fig. 8)

The plasmid pMCR611 (300 µg) [Miki, T. et al., Mol. Gen. Genet., 183, 25 (1981)] was digested with the restriction enzymes BamHI and PstI (Takara Shuzo). Agarose gel (1%) electrophoresis gave a 1.25 kbp DNA fragment containing the rec A promoter portion, which fragment was eluted from the gel [McDonell, M. W. et al., J. Mol. Biol., 110, 119 (1977)]. This DNA fragment was purified by phenol extraction, ether extraction and ethanol precipitation, then digested with the restriction enzyme HaeIII (Takara Shuzo), and fractionated by 1.5% agarose gel electrophoresis to give a 300 b DNA fragment containing the rec A promoter portion. The fragment was purified in the same manner as mentioned above. This DNA fragment was further digested with the restriction enzyme HpaII (Takara Shuzo). The digestion product was subjected to 8% acrylamide gel (acrylamide:bisacrylamide=19:1) electrophoresis so as to isolate the 148 b DNA fragment covering the sequence from −129 to 19 with the RNA transcription startpoint on the rec A promoter being counted as 1 as reported by Sancar et al. [Proc. Natl. Acad. Sci. USA, 77, 2611 (1980)]. The thus-fractionated fragment was purified in the same manner as above.

Separately, four oligonucleotides each containing a portion of the 3'-end of the rec A promoter, namely (1) dCGGTATTACCCGGC, (2) dATGACAGGAGTAAAAG, (3) dTCATGCCGGGTAATAG and (4) dGATCCTTTTACTCCTG, were chemically synthesized by the phosphotriester method [Proc. Natl. Acad. Sci. USA. 75, 5765 (1978)]. Of these oligonulceotides, (2) and (3) were phosphorylated at the 5'-end using polynucleotide kinase [New England Biolabs (NEB)] and ATP. Equal amounts (0.5 µg) of the four oligonucleotides, namely oligonucleotides (1) and (4) and 5'-phosphorylated oligonucleotides (2) and (3), were mixed and, using T4 DNA ligase, they were ligated to one another by maintaining the mixture in 50 µl of reaction medium (66 mM Tris-HCl, pH 7.6, 6.6 mM MgCl₂, 10 mM dithiothretitol, 1 mM ATP, 300 units T4 DNA ligase) at 14°C for 16 hours. The reaction product was fractionated by 10% acrylamide gel (acrylamide:bisacrylamide = 19.1) electrophoresis. The about 30 b DNA fragment, a ligation product of the oligonucleotides, was isolated and purified in the same manner as mentioned above.

In the next place, the above-mentioned 148 b DNA fragment and the 30 b DNA fragment were ligated together using T4 DNA ligase, The reaction product was fractionated by 8% acrylamide gel electrophoresis. The 178 b DNA fragment which thus formed was isolated and purified. The cohesive termini of this DNA fragment were rendered blunt by contacting the DNA fragment with DNA polymerase I large fragment [Bethesda Research Laboratroies (BRL)] in 30 µl of reaction mixture (40 mM potassium phosphate buffer, pH 7.5, 6.6 mM MgCl₂, 1 mM 2-mercaptoethanol, 20 µM dATP, 20 µM dGTP, 20 µM dCTP, 20 µM dTTP, 2 units DNA polymerase I large fragment) at 20°C for 30 minutes. The product DNA was purified by phenol extraction and ethanol precipitation and thereto was joined the BamHI linker dCCGGATCCGG (BRL) phosphorylated at the 5'-end, using T4 DNA ligase. The product was digested with the restriction enzyme BamHI and inserted into the pBR322 DNA BamHI digestion product. The resultant recombinant DNA was used for transformation of Escherichia coli strain 294. Amplicillin-resistant, tetracycline-sensitive transformants were selected and plasmid DNAs were isolated therefrom according to the method of Birnboim-Doly (vide supra). A plasmid which gave a 185 b DNA fragment upon digestion with the restriction enzyme BamHI was selected. At the same time, the base sequence of the 185 b DNA fragment thus cut out was determined according to the dideoxynucleotide synthetic chain termination method (vide supra). In this manner, there was obtained the product of ligation of the 148 b DNA fragment from the 5'-end portion of the rec A promoter with the BamHI linker as joined together in the expected manner. Its nucleotide sequence was as follows:

```
          −130           −120           −110           −100
           |              |              |              |
          GATCCGGCGGCGGGAATGCTTCAGCGGCGACCGTGATGCGG
                GCCGCCGCCCTTACGAAGTCGCCGCTGGCACTACGCC

           −90            −80            −70            −60
            |              |              |              |
          TGCGTCGTCAGGCTACTGCGTATGCTTGCAGACCTTGTGGC
          ACGCAGCAGTCCGATGACGCATACGAACGTCTGGAACACCG

           −50            −40            −30            −20
            |              |              |              |
          AACAATTTCTACAAAACACTTGATACTGTATGAGCATACAG
          TTGTTAAAGATGTTTTGTGAACTATGACATACTCGTATGTC

           −10             1      ·      10             20
            |              |              |              |
          TATAATTGCTTCAACAGAACATATTGACTATCCGGTATTAC
          ATATTAACGAAGTTGTCTTGTATAACTGATAGGCCATAATG

           30             40
            |              |
          CCGGCATGACAGGAGTAAAAG
          GGCCGTACTGTCCTCATTTTCCTAG
```

As compared with the report of Sancar, A et al., the above sequence contains one more base pair ($\frac{A}{T}$) at −121.

A pBR322 plasmid with this DNA fragment inserted therein with the directionality of the rec A promoter coinciding with that of the ampicillin resistance-offering β-lactamase gene was named pREC3.

(2) Construction of rec A promoter-containing expression vector (cf. Fig. 9)

(i) Expression vector with insert at restriction enzyme BamHI recognition site

The expression vector plasmid pREC3 containing the insert at the restriction enzyme BamHI recognition site was partially digested with the restriction enzyme BamHI. The resultant cohesive termini of the plasmid DNA cleaved at one site of the plasmid were rendered blunt according to the method described in Example 6-(1) using DNA polymerase I large fragment and rejoined together using T4 DNA ligase. The resultant DNA was used for transformation of *Escherichia coli* strain 294. Ampicillin-resistant transformants were selected, and plasmid DNAs were isolated therefrom according to the method of Birnboim-Doly (alkali extraction method). A plasmid in which the BamHI site on the 5'-end (upstream) of the rec A promoter was eliminated from the restriction enzyme cleavage pattern was selected and named pREC 33.

(ii) Expression vector with insert at restriction enzyme ClaI recognition site

The plasmid pREC3 was digested with the restriction enyzme BamHI, followed by 6% acrylamide gel electrophoresis, which gave a 185 b rec A promoter unit DNA. The cohesive termini of this DNA were rendered blunt with S1 nuclease (BRL) in 100 µl of reaction mixture (0.5 µg DNA, 0.03 M sodium acetate buffer, pH 4.5, 0.3 M NaCl, 4.5 mM $ZnCl_2$, 3 units S1 nuclease) at 22°C for 30 minutes and then the DNA was purified by phenol extraction and ethanol precipitation. The ClaI linker dCATCGATG (chemically synthesized by the phosphotriester method) was phosphorylated at the 5'-end and ligated with the above DNA using T4 DNA ligase. The ligation product was digested with the restriction enzyme ClaI (Boehringer) and ligated with the pBR322 DNA digested with the same enzyme. The ligation product was used for transformation of *Escherichia coli* strain 294. Ampicillin-resistant transformants were selected. Plasmid DNAs were isolated from these transformants according to the method of Birnboim-Doly (vide supra). Based on the restriction enzyme cleavage pattern, a plasmid (pREC103) containing the rec A promoter in the same directionality as that of the β-lactamase gene and a plasmid (pREC113) containing the rec A promoter in the directionality opposite to the β-lactamase gene were selected. In pREC113, the rec A promoter is directed toward the tetracycline resistance gene, so that the transformant carrying this plasmid is partially resistant to tetracycline.

pREC103 and pREC113 were partially cleaved with the restriction enzyme ClaI and, following the procedure of Example 6-(2-i), plasmids with the ClaI recognition site on the 5'-end side of (upstream from) the rec A promoter eliminated were selected and named pREC 144 and pREC136, respectively.

(iii) Expression vector with insert at restriction enzyme EcoRI recognition site

The plasmid pREC3 was digested with the restriction enzyme BamHI and, following the procedure of Example 6-(2-ii), a rec A promoter unit DNA was isolated and rendered blunt-ended. This DNA was ligated with the EcoRI linker dGGAATTCC (BRL) phosphorylated at the 5'-end, using T4 DNA ligase, followed by

13

digestion with the restriction enzyme EcoRI (Takara Shuzo) and ligation with the pBR322 DNA digested with the same enzyme using T4 DNA ligase. The ligation product was used for transformation of *Escherichia coli* strain 294, and ampicillin- and tetracycline- resistant transformants were selected. Plasmid DNAs were isolated from these transformants according to the method of Birnboim-Doly (vide supra) and, based on the restriction enzyme cleavage pattern, a plasmid (pREC206) with the rec A promoter agreeing in directionality with the β-lactamase gene and one (pREC207) with the rec A promoter contrary in directionality to said gene were selected.

(3) Construction of expression vector with rec A promoters connected in series (cf. Fig. 10)

The plasmid pREC144 constructed to Example 6-(2) was double-digested with the restriction enzymes ClaI and PstI, followed by 1.2% agarose gel electrophoresis. Then, a 3.8 kbp DNA fragment containing the rec A promoter portion was isolated and purified according to the same method as described in Example 6-(1).

The plasmid pREC103 constructed in Example 6-(2) was separately digested with the restriction enzyme PstI and further digested partially with the restriction enzyme ClaI, and a 0.96 kbp DNA fragment containing the rec A promoter portion was isolated according to the above-mentioned method. This fragment was ligated with the above-mentioned 3.8 kbp DNA fragment with T4 DNA ligase to give an expression plasmid, pREC2102, with two rec A promoters connected in series. With this plasmid, a gene desired to be expressed can be inserted thereinto at the ClaI recognition site.

Separately, the plasmid pREC206 constructed in Example 2 was double-digested with the restriction enzymes ClaI and PstI, and a 0.96 kbp DNA fragment containing the rec A promoter portion was isolated according to the above-mentioned method. This was ligated with the above-mentioned 3.8 kbp DNA fragment using T4 DNA ligase to give an expression plasmid, pREC2201, containing two rec A promoters connected in series. With this plasmid, a gene desired to be expressed can be inserted thereinto at the EcoRI recognition site.

(4) Construction of human immunoglobulin E H-chain gene expression plasmid (cf. Fig. 11)

The plasmid pGETtrp302 with the human immunoglobulin E H-chain gene inserted therein downstream from the tryptophan promoter obtained in Example 3-(ii) was cleaved with the restriction enzyme ClaI. The resultant cohesive termini were rendered blunt by contacting with S1 nuclease (BRL) in 100 µl of reaction mixture (2 µg DNA, 0.03 M sodium acetate buffer, pH 4.5, 0.3 M NaCl, 4.5 mM ZnCl$_2$, 12 units S1 nuclease) at 22°C for 30 minutes. This DNA was purified by phenol extraction and ethanol precipitation and then ligated with the EcoRI linker dCTAGAATTCTAG (chemically synthesized by the phosphotriester method and phosphorylated at the 5'-end) using T4 DNA ligase. The ligation product was double-digested with the restriction enzymes EcoRI and PstI, and a 1.2 kb DNA fragment was isolated by the subsequent 1% agarose gel electrophoresis and purified according to the method described in Example 6-(1).

Separately, the plasmid pREC206 was digested with the restriction enzyme PstI and then further digested partially with the restriction enzyme EcoRI, the digestion product was subjected to 1% agarose gel electrophoresis, and a 3.8 kb DNA fragment was isolated and purified by the method described in Example 6-(1). This DNA was ligated with the aforementioned 1.2 kb human immunoglobulin E H-chain gene using T4 DNA ligase. The ligation product was used for transformation of *Escherichia coli* strain 294, and tetracycline-resistant transformants were selected. Plasmid DNAs were isolated from these transformants according to the method of Birnboim-Doly (vide supra) and, based on the restriction enzyme cleavage pattern, an expression plasmid, pGETrec301, with the rec A promoter and the human immunoglobulin E H-chain gene interconnectedly inserted therein was obtained. *Escherichia coli* 294/pGETrec301 has been deposited with the Institute for Fermentation, Osaka under deposit No. IFC-14287.

(5) Expression of human immunoglobulin E H-chain gene in *Escherichia coli* and isolation of human immunoglobulin E H-chain polypeptide

The transformant carrying the human immunoglobulin E H-chain gene expression plasmid pGETrec301 as obtained in Example 6-(4) was cultured in M-9 medium containing 1% glucose and 0.4% Casamino acids for 4 hours. Then, nalidixic acid was added in the concentration of 50 µg/ml, and incubation was continued for further 4 hours. Thereafter, cells were harvested and suspended in 1/50 volume of a solution containing 50 mM Tris-HCl, pH 8.0, 0.2 M NaCl, 0.01 M EDTA, 10% sucrose and 150 µg/ml lysozyme. The suspension was allowed to stand at 0°C for 50 minutes and then treated at 37°C for 2 minutes. Thereafter, the suspension was cooled back to 0°C and, after addition of 0.1% of Triton X100, subjected to sonication, followed by centrifugation at 15,000 rpm (Sorvall, SS34 rotor) for 30 minutes. The supernatant thus obtained had an immunoglobulin E activity of 1.1 µg/ml as measured by the RIST method (vide supra). The immunoglobulin E H-chain polypeptide can be isolated from this supernatant and purified as described in Example 5, that is by using an anti-human immunoglobulin E monoclonal antibody column.

**Claims for the Contracting States: BE, CH, DE, FR, GB, IT LI, NL, SE**

1. The polynucleotide having the sequence 831—1485 as shown in Figure 1.

2. The polynucleotide having the sequence 490—830 as shown in Figure 1, or a fragment thereof, linked to the 5' end of the polynucleotide of Claim 1.

3. The polynucleotide having the sequence 88—489 as shown in Figure 1, or a fragment thereof, linked to the 5' end of the polynucleotide of Claim 2.

4. The polynucleotide having the sequence 1—87 as shown in Figure 1, or a fragment thereof, linked to the 5' end of the polynucleotide of Claim 3.

5. The polynucelotide having ATG at the 5' end of the polynucleotide as claimed in any of claims 1 to 4 without any reading frame shift.

6. A plasmid which contains the polynucleotide having the sequence 831—1485 as shown in Figure 1.

7. A plasmid according to Claim 6, which contains the polynucleotide having the sequence 490—830, or a fragment thereof, linked to the 5' end of the polynucleotide of Claim 6.

8. A plasmid according to Claim 6, which contains the polynucleotide having the sequence 88—489 as shown in Figure 1, or a fragment thereof, linked to the 5' end of the polynucleotide of Claim 7.

9. A plasmid according to Claim 6, which contains the polynucleotide having the sequence 1—87 as shown in Figure 1, or a fragment thereof, linked to the 5' end of the polynucleotide of Claim 8.

10. A plasmid according to any of Claims 6 to 9, wherein it has ATG at the 5' end of the polynucleotide without any reading frame shift.

11. A plasmid according to any of Claims 6 to 10, wherein the polynucleotide is linked at a site downstream from a promoter.

12. A plasmid according to Claim 11, wherein the promoter is trp promoter.

13. A method of producing a plasmid containing a polynucelotide having the sequence 831—1485 as shown in Figure 1, which comprises:

(a) growing human immunoglobulin E-secreting cells

(b) isolating from the cells a messenger RNA coding for the human immunoglobulin E,

(c) synthesizing a single-stranded complementary DNA with a use of said messenger RNA and reverse transcriptase,

(d) converting said complementary DNA to a double-stranded DNA, and,

(e) inserting said double-stranded DNA into a plasmid, and if desired,

(f) transforming a host organism with said recombinant plasmid, and if desired,

(g) isolating a plasmid containing the objective DNA from the culture of said host organism, and if desired,

(h) excising out the cloned DNA from the plasmid, and if desired,

(i) connecting said cloned DNA at a site downstream from a promoter in a vehicle.

14. A transformant belonging to *Escherichia coli* which contains a plasmid containing the polynucleotide having the sequence 831—1485 as shown in Figure 1.

15. A transformant according to Claim 14, wherein the plasmid contains the polynucleotide having the sequence 490—1485 as shown in Figure 1.

16. A transformant according to Claim 14, wherein the plasmid contains the polynucleotide having the sequence 88—1485 as shown in Figure 1.

17. A transformant according to Claim 14, wherein the plasmid contains the polynucleotide having the sequence 1—1485 as shown in Figure 1.

18. The transformant according to Claim 14, which is *Escherichia coli* 294/pGETtrp104 (IFO-14284).

19. The transformant according to Claim 14, which is *Escherichia coli* 294/pGETtrp302 (IFO-14285).

20. The transformant according to Claim 14, which is *Escherichia coli* 294/pGETtrp410 (IFO-14286).

21. A method of producing a polypeptide of or equivalent in immunological or biological activities to the human immunoglobulin E H-chain, which comprises growing a transformant belonging to *Escherichia coli* which contains a plasmid containing the polynucleotide having the sequence 831—1485 as shown in Figure 1, accumulating said polypeptide and recovering the same.

22. A method according to Claim 21, wherein the transformant is *Escherichia coli* 294/pGETtrp104 (IFO-14284).

23. A method according to Claim 21, wherein the transformant is *Escherichia coli* 294/pGETtrp302 (IFO-14285).

24. A method according to Claim 21, wherein the transformant is *Escherichia coli* 294/pGETtrp410 (IFO-14286).

**Claims for the Contracting State: AT**

1. A method of producing a plasmid containing a polynucleotide having the sequence 831—1485 as shown in Figure 1, which comprises:

(a) growing human immunoglobulin E-secreting cells,

(b) isolating from the cells a messenger RNA coding for the human immunoglobulin E,

(c) synthesizing a single-stranded complementary DNA with a use of said messenger RNA and reverse transcriptase,

(d) converting said complementary DNA to a double-stranded DNA, and

(e) inserting said double-stranded DNA into a plasmid, and if desired,

(f) transforming a host organism with said recombinant plasmid, and if desired,

(g) isolating a plasmid containing the objective DNA from the culture of said host organism, and if desired,

(h) excising out the cloned DNA from the plasmid, and if desired,

(i) connecting said cloned DNA at a site downstream from a promoter in a vehicle.

2. Method according to Claim 1, wherein the plasmid contains the polynucleotide having the sequence 490—830, or a fragment thereof, linked to the 5' end of the polynucleotide of Claim 1.

3. Method according to Claim 1, wherein the plasmid contains the polynucleotide having the sequence 88—489 as shown in Figure 1, or a fragment thereof, linked to the 5' end of the polynucleotide of Claim 2.

4. Method according to Claim 1, wherein the plasmid contains the polynucleotide having the sequence 1—87 as shown in Figure 1, or a fragment thereof, linked to the 5' end of the polynucleotide of Claim 3.

5. A method according to any of Claims 1 to 4, wherein the plasmid has ATG at the 5' end of the polynucleotide without any reading frame shift.

6. A method according to any of Claims 1 to 5, wherein the plasmid containing the polynucleotide is linked at a site downstream from a promoter.

7. A method according to Claim 6, wherein the promoter is trp promoter.

8. A method of producing a polypeptide of or equivalent in immunological or biological activities to the human immunoglobulin E H-chain, which comprises growing a transformant belonging to *Escherichia coli* which contains a plasmid containing the polynucleotide having the sequence 831—1485 as shown in Figure 1, accumulating said polypeptide and recovering the same.

9. A method according to Claim 8, wherein the transformant is *Escherichia coli* 294/pGETtrp104 (IFO-14284).

10. A method according to Claim 8, wherein the transformant is *Escherichia coli* 294/pGETtrp302 (IFO-14285).

11. A method according to Claim 8, wherein the transformant is *Escherichia coli* 294/pGETtrp410 (IFO-14286).

12. A method according to Claim 8, wherein the polynucleotide having the sequence 490—830 as shown in Figure 1, or a fragment thereof, is linked to the 5' end of the polynucleotide of Claim 8.

13. A method according to Claim 8, wherein the polynucleotide having the sequence 88—489 as shown in Figure 1, or a fragment thereof, is linked to the 5' end of the polynucleotide of Claim 12.

14. A method according to Claim 8, wherein the polynucleotide having the sequence 1—87 as shown in Figure 1, or a fragment thereof, is linked to the 5' end of the polynucleotide of Claim 13.

15. A method according to any of Claims 8 and 12 to 14, wherein the polynucleotide has ATG at the 5' end of the polynucleotide without any reading frame shift.

16. A transformant belonging to *Escherichia coli* which contains a plasmid containing the polynucleotide having the sequence 831—1485 as shown in Figure 1.

17. A transformant according to Claim 16, wherein the plasmid contains the polynucleotide having the sequence 490—1485 as shown in Figure 1.

18. A transformant according to Claim 16, wherein the plasmid contains the polynucleotide having the sequence 88—1485 as shown in Figure 1.

19. A transformant according to Claim 16, wherein the plasmid contains the polynucleotide having the sequence 1—1485 as shown in Figure 1.

20. The transformant according to Claim 16, which is *Escherichia coli* 294/pGETtrp104 (IFO-14284).

21. The transformant according to Claim 16, which is *Escherichia coli* 294/pGETtrp302 (IFO-14285).

22. The transformant according to Claim 16, which is *Escherichia coli* 294/pGETtrp410 (IFO-14286).

**Patentansprüche für die Vertragsstaaten: BE, CH, DE, FR, GB, IT, LI, NL, SE**

1. Das Polynucleotid mit der in Fig. 1 dargestellten Sequenz 831—1485.

2. Das Polynucleotid mit der in Fig. 1 dargestellten Sequenz 490—830 oder ein Fragment desselben, das an das 5'-Ende des Polynucleotides nach Anspruch 1 gebunden ist.

3. Das Polynucleotid mit der in Fig. 1 dargestellten Sequenz 88—489 oder ein Fragment desselben, das an das 5'-Ende des Polynucleotides nach Anspruch 2 gebunden ist.

4. Das Polynucleotid mit der in Fig. 1 dargestellten Sequenz 1—87 oder ein fragment desselben, das an das 5'-Ende des Polynucleotides nach Anspruch 3 gebunden ist.

5. Das Polynucleotid mit ATG am 5'-Ende des Polynucleotides nach einem der Ansprüche 1 bis 4, ohne reading frame shift.

6. Ein Plasmid, das das Polynucleotid mit der in Fig. 1 dargestellten Sequenz 831—1485 enthält.

7. Ein Plasmid nach Anspruch 6, das das Polynucleotid mit der Sequenz 490—830 oder ein Fragment desselben, gebunden an das 5'-Ende des Polynucleotides nach Anspruch 6, enthält.

8. Ein Plasmid nach Anspruch 6, das das Polynucleotid mit der in Fig. 1 dargestellten Sequenz 88—489 oder ein Fragment desselben, gebunden an das 5'-Ende des Polynucleotides nach Anspruch 7 enthält.

9. Ein Plasmid nach Anspruch 6, das das Polynucleotid mit der in Fig. 1 dargestellten Sequenz 1—87 oder ein Fragment desselben, gebunden an das 5'-Ende des Polynucleotides nach Anspruch 8 enthält.

10. Ein Plasmid nach einem der Ansprüche 6 bis 9, das ATG am 5'-Ende des Polynucleotides ohne reading frame shift aufweist.

11. Ein Plasmid nach einem der Ansprüche 6 bis 10, worin das Polynucleotid an einer Stelle stromabwärts von einem Promotor gebunden ist.

12. Das Plasmid nach Anspruch 11, worin der Promotor ein trp-Promotor ist.

13. Verfahren zur Herstellung eines Plasmids, das ein Nucleotid mit der in Fig. 1 dargestellten Sequenz 831—1485 enthält, umfassend:

(a) Kultivieren menschliches Immunoglobulin E absondernder Zellen.

(b) Isolieren einer Boten-RNA aus den Zellen, die für das menschliche Immunoglobulin E kodiert.

(c) Synthese einer elastrangigen komplementären DNA unter Verwendung der genannten Boten-RNA und Umkehr-Transcriptase.

(d) Umwandlung der genannten komplementären DNA in eine doppelstrangige DNA, und

(e) Einsetzen der genannten doppelstrangigen DNA in ein Plasmid, und nach Bedarf,

(f) Umwandlung eines Wirtsorganismus mit dem genannten rekombinanten Plasmid, und nach Bedarf,

(g) Isolieren eines Plasmides, das die gegenständliche DNA enthält, aus der Kultur des genannten Wirtsorganismus, und nach Bedarf,

(h) Exzision der geklonten DNA aus dem Plasmid, und nach Bedarf,

(i) Verbinden der genannten geklonten DNA an eine Stelle stromabwärts von einem Promotor in einem Vehiculum.

14. Ein Transformant der Gattung Escherichia coli, der ein Plasmid enthält, das das Polynucleotid mit der in Fig. 1 dargestellten Sequenz 831—1485 enthält.

15. Ein Transformant nach Anspruch 14, worin das Plasmid das Polynucleotid mit der in Fig. 1 dargestellten Sequenz 490—1485 enthält.

16. Ein Transformant nach Anspruch 14, worin das Plasmid das Polynucleotid mit der in Fig. 1 dargestellten Sequenz 88—1485 enthält.

17. Ein Transformant nach Anspruch 14, worin das Plasmid das Polynucleotid mit der in Fig. 1 dargestellten Sequenz 1—1485 enthält.

18. Der Transformant nach Anspruch 14, der Escherichia coli 294/pGETtrp104 (IFO-14284) ist.

19. Der Transformant nach Anspruch 14, der Escherichia coli 294/pGETtrp302 (IFO-14285) ist.

20. Der Transformant nach Anspruch 14, der Escherichia coli 294/pGETtrp410 (IFO-14286) ist.

21. Verfahren zur Herstellung eines Polypeptides der menschlichen Immunoglobulin E H-Kette oder eines Polypeptides, das von äquivalenter immunologischer oder biologischer Aktivität in bezug auf die menschliche Immunoglobulin E H—Kette ist, umfassend Kultivieren eines Transformanten der Gattung Escherichia coli, der ein Plasmid enthält, das das Polynucleotid mit der in Fig. 1 dargestellten Sequenz 831—1485 enthält, Ansammeln des genannten Polypeptides und Gewinnen desselben.

22. Verfahren nach Anspruch 21, worin der Transformant Escherichia coli 294/pGETtrp104 (IFO-14284) ist.

23. Verfahren nach Anspruch 21, worin der Transformant Esherichia coli 294/pGETtrp302 (IFO-14285) ist.

24. Verfahren nach Anspruch 21, worin der Transformant Escherichia coli 294/pGETtrp410 (IFO-14286) ist.

**Patentansprüche für den Vetragsstaat: AT**

1. Verfahren zur Herstellung eines Plasmids, das ein Polynucleotid mit der in Fig. 1 dargestellten Sequenz 831—1485 enthält, umfassend:

(a) Kultivieren meschliches Immunoglobulin E absondernder Zellen,

(b) Isolieren einer Boten-RNA, die für das menschliche Immunoglobulin E kodiert, aus den Zellen,

(c) Synthetisieren einer einstrangigen, komplementären DNA unter Verwendung der genannten Boten-RNA und Umkehr-Transcriptase,

(d) Umwandeln der genannten komplementären DNA in eine duppelstrangige DNA, und

(e) Einfügen der genannten doppelstrangigen DNA in ein Plasmid, und nach Bedarf,

(f) Umwandeln eines Wirtsorganismus mit dem genannten rekombinanten Plasmid, und nach Bedarf,

(g) Isolieren eines Plasmides, das die gegenständliche DNA enthält, aus der Kultur des genannten Wirtsorgansimus, und nach Bedarf,

(h) Exzisteren der geklonten DNA aus dem Plasmid und nach Bedarf,

(i) Verbinden der genannten geklonten DNA an einer Stelle stromabwärts von einem Promoter in einem Vehiculum.

2. Verfahren nach Anspruch 1, worin das Plasmid das Polynucleotid mit der Sequenz 490—830 oder ein Fragment desselben enthält, das an das 5'-Ende des Polynucleotides nach Anspruch 1 gebunden ist.

3. Verfahren nach Anspruch 1, worin das Plasmid das Polynucleotid mit der in Fig. 1 dargestellten Sequenz 88—489 oder ein Fragment desselben enthält, das an das 5'-Ende des Polynucleotides nach Anspruch 2 gebunden ist.

4. Verfahren nach Anspruch 1, worin das Plasmid das Polynucleotid mit der in Fig. 1 dargestellten Sequenz 1—87 oder ein Fragment desselben enthält, das an das 5'-Ende des Polynucleotides nach Anspruch 3 gebunden ist.

5. Verfahren nach einem der Ansprüche 1 bis 4, worin das Plasmid ATG am 5'-Ende des Polynucleotides ohne reading frame shift aufweist.

6. Verfahren nach einem der Ansprüche 1 bis 5, worin das das Polynucleotide enthaltende Plasmid an einer Stelle stromabwärts von einem Promotor gebunden ist.

7. Verfahren nach Anspruch 6, worin der Promotor ein trp-Promotor ist.

8. Verfahren zur Herstellung eines Polypeptides der menschlichen Immunoglobulin E H-Kette oder eines Polypeptides, das dieser in immunologischer oder biologischer Aktivität äquivalent ist, umfassend das Kultivieren eines Transformanten der Gattung Escherichia coli, der ein Plasmid enthält, das das Polynucleotid mit der in Fig. 1 dargestellten Sequenz 831—1485 enthält, Ansammeln des genannten Polypeptides und Gewinnen desselben.

9. Verfahren nach Anspruch 8, worin der Transformant Escherichia coli 294/pGETtrp104 (IFO-14284) ist.

10. Verfahren nach Anspruch 8, worin der Transformant Escherichia coli 294/pGETtrp302 (IFO-14285) ist.

11. Verfahren nach Anspruch 8, worin der Transformant Escherichia coli 294/pGETtrp410 (IFO-14286) ist.

12. Verfahren nach Anspruch 8, worin das Polynucleotid mit der in Fig. 1 dargestellten Sequenz 490—830 oder ein Fragment desselben an das 5'-Ende des Polynucleotides nach Anspruch 8 gebunden ist.

13. Verfahren nach Anspruch 8, worin das Polynucleotid mit der in Fig. 1 dargestellten Sequenz 88—489 oder ein Fragment desselben an das 5'-Ende des Polynucleotides nach Anspruch 12 gebunden ist.

14. Verfahren nach Anspruch 8, worin das Polynucleotid mit der in Fig. 1 dargestellten Sequenz 1—87 oder ein Fragment desselben an das 5'-Ende des Polynucleotides nach Anspruch 13 gebunden ist.

15. Verfahren nach einem der Ansprüche 8 und 12 bis 14, worin das Polynucleotid ATG am 5'-Ende des Polynucleotids ohne reading frame shift aufweist.

16. Ein Transformant der Gattung Escherichia coli, der ein Plasmid enthält, das ein Polynucleotid mit der in Fig. 1 dargestellten Sequenz 831—1485 enthält.

17. Ein Tansformant nach Anspruch 16, worin das Plasmid das Polynucleotid mit der in Fig. 1 dargestellten Sequenz 490—1485 enthält.

18. Ein Transformant nach Anspruch 16, worin das Plasmid das Polynucleotid mit der in Fig. 1 dargestellten Sequenz 88—1485 enthält. ·

19. Ein Transformant nach Anspruch 16, worin das Plasmid das Polynucleotid mit der in Fig. 1 dargestellten Sequenz 1—1485 enthält.

20. Der Transformant nach Anspruch 16, der Escherichia coli 294/pGETtrp104 (IFO-14284) ist.

21. Der Transformant nach Anspruch 16, der Escherichia coli 294/pGETtrp302 (IFO-14285) ist.

22. Der Transformant nach Anspruch 16, der Escherichia coli 294/pGETtrp410 (IFO-14286) ist.

**Revendications pour les Etats Contractants: BE, CH, DE, FR, GB, IT, LI, NL, SE**

1. Polynucléotide possédant la séquence 831—1485 représentée sur la figure 1.

2. Polynucléotide possédant la séquence 490—830 représentée sur la figure 1, ou un fragment de celle-ci, lié à l'extrémité 5' du polynucléotide de la revendication 1.

3. Polynucléotide possédant la séquence 88—489 représentée sur la figure 1, ou un fragment de celle-ci, lié à l'extrémité 5' du polynucléotide de la revendication 2.

4. Polynucléotide possédant la séquence 1—87 représentée sur la figure 1, ou un fragment de celle-ci, lié à l'extrémité 5' du polynucléotide de la revendication 3.

5. Polynucléotide possédant ATG à l'extrémité 5' du polynucléotide selon l'une quelconque des revendications 1 à 4, sans décalage de phase de lecture.

6. Plasmide qui contient le polynucléotide possédant la séquence 831—1485 représentée sur la figure 1.

7. Plasmide selon la revendication 6, qui contient le polynucléotide possédant la séquence 490—830, ou un fragment de celle-ci, lié à l'extrémité 5' du polynucléotide de la revendication 6.

8. Plasmide selon la revendication 6, qui contient le polynucléotide possédant la séquence 88—489 représentée sur la figure 1, ou un fragment de celle-ci, lié à l'extrémité 5' du polynucléotide de la revendication 7.

9. Plasmide selon la revendication 6, qui contient le polynucléotide possédant la séquence 1—87 représentée sur la figure 1, ou un fragment de celle-ci, lié à l'extrémité 5' du polynucléotide de la revendication 8.

10. Plasmide selon l'une quelconque des revendications 6 à 9, qui possède ATG à l'extrémité 5' du polynucléotide sans aucun décalage de phase de lecture.

11. Plasmide selon l'une quelconque des revendications 6 à 10, dans lequel le polynucléotide est lié à un site en aval d'un promoteur.

12. Plasmide selon la revendication 11, dans lequel le promoteur est le promoteur trp.

13. Procédé de production d'un plasmide contenant un polynucléotide possédant la séquence 831—1485 représentée sur la figure 1, qui comprend les étapes qui consistent:

18

**0 102 634**

(a) à cultiver des cellules sécrétant l'immunoglobuline E humaine,

(b) à isoler des cellules un ARN messager codant pour l'immunoglobuline E humaine,

(c) à synthétiser un ADN complémentaire à mono-brin à l'aide de l'ARN messager et d'une transcriptase inverse,

(d) à transformer cet ADN complémentaire en un ADN à double brin, et

(e) à insérer l'ADN à double brin dans un plasmide, et, le cas échéant,

(f) à transformer un organisme hôte à l'aide du plasmide recombinant, et, le cas échéant,

(g) à isoler de la culture de l'organisme hôte un plasmide contenant l'ADN cible, et, le cas échéant,

(h) à séparer par excision l'ADN cloné d'avec le plasmide, et, le cas échéant,

(i) à relier l'ADN cloné à un site en aval d'un promoteur dans un véhicule.

14. Transformant appartenant à *Escherichia coli*, qui contient un plasmide contenant le polynucléotide possédant la séquence 831—1485 représentée sur la figure 1.

15. Transformant selon la revendication 14, dans lequel le plasmide contient le polynucléotide possédant la séquence 490—1485 représentée sur la figure 1.

16. Transformant selon la revendication 14, dans lequel le plasmide contient le polynucléotide possédant la séquence 88—1485 représentée sur la figure 1.

17. Transformant selon la revendication 14, dans lequel le plasmide contient le polynucléotide possédant la séquence 1—1485 représentée sur la figure 1.

18. Transformant selon la revendication 14, qui est *Escherichia coli* 294/pGETtrp104 (IFO-14284).

19. Transformant selon la revendication 14, qui est *Escherichia coli* 294/pGETtrp302 (IFO-14285).

20. Transformant selon la revendication 14, qui est *Escherichia coli* 294/pGETtrp410 (IFO-14286).

21. Procédé de production d'un polypeptide de la chaîne H de l'immunoglobuline E humaine, ou d'un équivalent de cette chaîne pour les activités immunologiques ou biologiques, qui comprend la culture d'un transformant appartenant à *Escherichia coli* qui contient un plasmide contenant le polynucléotide possédant la séquence 831—1485 représentée sur la figure 1, l'accumulation de ce polypeptide et sa récupération.

22. Procédé selon la revendication 21, dans lequel le transformant est *Escherichia coli* 294/pGETtrp104 (IFO-14284).

23. Procédé selon la revendication 21, dans lequel le transformant est *Escherichia coli* 294/pGETtrp302 (IFO-14285).

24. Procédé selon la revendication 21, dans lequel le transformant est *Escherichia coli* 294/pGETtrp410 (IFO-14286).

**Revendications pour l'Etat Contractant: AT**

1. Procédé de production d'un plasmide contenant un polynucléotide possédant la séquence 831—1485 représentée sur la figure 1, qui comprend les étapes qui consistent:

(a) à cultiver des cellules sécrétant l'immunoglobuline E humaine,

(b) à isoler des cellules un ARN messager codant pour l'immunoglobuline E humaine,

(c) à synthétiser un ADN complémentaire à mono-brin à l'aide de l'ARN messager et d'une transcriptase inverse,

(d) à transformer cet ADN complémentaire en un ADN à double brin, et

(e) à insérer l'ADN à double brin dans un plasmide, et, le cas échéant,

(f) à transformer un organisme hôte à l'aide du plasmide recombinant, et, le cas échéant,

(g) à isoler de la culture de l'organisme hôte un plasmide contenant l'ADN cible, et, le cas échéant,

(h) à séparer par excision l'ADN cloné d'avec le plasmide, et, le cas échéant,

(i) à relier l'ADN cloné à un site en aval d'un promoteur dans un véhicule.

2. Procédé selon la revendication 1, dans lequel le plasmide contient le polynucléotide possédant la séquence 490—830, ou un fragment de celle-ci, lié à l'extrémité 5' du polynucléotide de la revendication 1.

3. Procédé selon la revendication 1, dans lequel le plasmide contient le polynucléotide possédant la séquence 88—489 représentée sur la figure 1, ou un fragment de celle-ci, lié à l'extrémité 5' du polynucléotide de la revendication 2.

4. Procédé selon la revendication 1, dans lequel le plasmide contient le polynucléotide possédant la séquence 1—87 représentée sur la revendication 1, ou un fragment de celle-ci, lié à l'extrémité 5' du polynucléotide de la revendication 3.

5. Procédé selon l'une quelconque des revendications 1 à 4, dans lequel le plasmide possède ATG à l'extrémité 5' du polynucléotide sans aucun décalage de phase de lecture.

6. Procédé selon l'une quelconque des revendications 1 à 5, dans lequel le plasmide contenant le polynucléotide est lié à un site en aval d'un promoteur.

7. Procédé selon la revendication 6, dans lequel le promoteur est le promoteur trp.

8. Procédé de production d'un polypeptide de la chaîne H de l'immunoglobuline E humaine, ou d'un équivalent de cette chaîne pour les activités immunologiques ou biologiques, qui comprend la culture d'un transformant appartenant à *Escherichia coli* qui contient un plasmide contenant le polynucléotide possédant la séquence 831—1485 représentée sur la figure 1, l'accumulation de ce polypeptide et sa récupération.

19

9. Procédé selon la revendication 8, dans lequel le transformant est *Escherichia coli* 294/pGETtrp104 (IFO-14284).

10. Procédé selon la revendication 8, dans lequel le transformant est *Escherichia coli* 294/pGETtrp302 (IFO-14285).

11. Procédé selon la revendication 8, dans lequel le transformant est *Escherichia coli* 294/pGETtrp410 (IFO-14286).

12. Procédé selon la revendication 8, dans lequel le polynucléotide possédant la séquence 490—830 représentée sur la figure 1, ou un fragment de celle-ci, est lié à l'extrémité 5' du polynucléotide de la revendication 8.

13. Procédé selon la revendication 8, dans lequel le polynucléotide possédant la séquence 88—489 représentée sur la figure 1, ou un fragment de celle-ci, est lié à l'extrémité 5' du polynucléotide de la revendication 12.

14. Procédé selon la revendication 8, dans lequel le polynucléotide possédant la séquence 1—87 représentée sur la figure 1, ou un fragment de celle-ci, est lié à l'extrémité 5' du polynucléotide de la revendication 13.

15. Procédé selon l'une quelconque des revendications 8 et 12 à 14, dans lequel le polynucléotide possède ATG à l'extrémité 5' du polynucléotide sans décalage de phase de lecture.

16. Transformant appartenant à *Escherichia coli*, qui contient un plasmide contenant le polynucléotide possédant la séquence 831—1485 représentée sur la figure 1.

17. Transformant selon la revendication 16, dans lequel le plasmide contient le polynucléotide possédant la séquence 490—1485 représentée sur la figure 1.

18. Transformant selon la revendication 16, dans lequel le plasmide contient le polynucléotide possédant la séquence 88—1485 représentée sur la figure 1.

19. Transformant selon la revendication 16, dans lequel le plasmide contient le polynucléotide possédant la séquence 1—1485 représentée sur la figure 1.

20. Transformant selon la revendication 16, qui est *Escherichia coli* 294/pGETtrp104 (IFO-14284).

21. Transformant selon la revendication 16, qui est *Escherichia coli* 294/pGETtrp302 (IFO-14285).

22. Transformant selon la revendication 16, qui est *Escherichia coli* 294/pGETtrp410 (IFO-14286).

## Figure 1

```
            10        20        30        40        50

           *         *    .     *         *         *
AGATTTCAGGGCAGGGTCACCATGACCAGAGACGCGTCCTTCAGTACAGC      50
TCTAAAGTCCCGTCCCAGTGGTACTGGTCTCTGCGCAGGAAGTCATGTCG

           *         *         *         *         *
CTACATGGACCTGAGAAGTCTGAGATCTGACGACTCGGCCGTGTTTTACT      100
GATGTACCTGGACTCTTCAGACTCTAGACTGCTGAGCCGGCACAAAATGA

           *         *         *         *         *
GTGCGAAAAGTGACCCTTTTTGGAGTGATTATTATAACTTTGACTACTCG      150
CACGCTTTTCACTGGGAAAAACCTCACTAATAATATTGAAACTGATGAGC

           *         *         *         *         *
TACACTTTGGACGTCTGGGGCCAAGGGACCACGGTCACCGTCTCCTCAGC      200
ATGTGAAACCTGCAGACCCCGGTTCCCTGGTGCCAGTGGCAGAGGAGTCG

           *         *    .    *         *         *
CTCCACACAGAGCCCATCCGTCTTCCCCCTTGACCCGCTGCTGCAAAAACA      250
GAGGTGTGTCTCGGGTAGGCAGAAGGGGAACTGGGCGACGACGTTTTTGT

           *         *         *         *         *
TTCCCTCCAATGCCACCTCCGTGACTCTGGGCTGCCTGGCCACGGGCTAC      300
AAGGGAGGTTACGGTGGAGGCACTGAGACCCGACGGACCGGTGCCCGATG

           *         *         *         *         *
TTCCCGGAGCCGGTGATGGTGACCTGGGACACAGGCTCCCTCAACGGGAC      350
AAGGGCCTCGGCCACTACCACTGGACCCTGTGTCCGAGGGAGTTGCCCTG

           *         *     .   *       . .*    .    *
AACTATGACCTTACCAGCCACCACCCTCACGCTCTCTGGTCACTATGCCA      400
TTGATACTGGAATGGTCGGTGGTGGGAGTGCGAGAGACCAGTGATACGGT

           *         *         *         *         *
CCATCAGCTTGCTGACCGTCTCGGGTGCGTGGGCCAAGCAGATGTTCACC      450
GGTAGTCGAACGACTGGCAGAGCCCACGCACCCGGTTCGTCTACAAGTGG

           *         *         *         *       * .
TGCCGTGTGGCACACACTCCATCGTCCACAGACTGGGTCGACAACAAAAC      500
ACGGCACACCGTGTGTGAGGTnGCAGGTGTCTGACCCAGCTGTTGTTTTG
```

1

Figure 1

```
         10        20        30        40        50
          *         *         *         *         *
CTTCAGCGTCTGCTCCAGGGACTTCACCCCGCCCACCGTGAAGATCTTAC      550
GAAGTCGCAGACGAGGTCCCTGAAGTGGGGCGGGTGGCACTTCTAGAATG


          *         *         *         *         *
AGTCGTCCTGCGACGGCGGCGGGCACTTCCCCCCGACCATCCAGCTCCTG      600
TCAGCAGGACGCTGCCGCCGCCCGTGAAGGGGGGCTGGTAGGTCGAGGAC


          *         *         *         *         *
TGCCTCGTCTCTGGGTACACCCCAGGGACTATCAACATCACCTGGCTGGA      650
ACGGAGCAGAGACCCATGTGGGGTCCCTGATAGTTGTAGTGGACCGACCT


          *         *         *         *         *
GGACGGGCAGGTCATGGACGTGGACTTGTCCACCGCCTCTACCACGCAGG      700
CCTGCCCGTCCAGTACCTGCACCTGAACAGGTGGCGGAGATGGTGCGTCC


          *          *         *         *         *
AGGGTGAGCTGGCCTCCACACAAAGCGAGCTCACCCTCAGCCAGAAGCAC      750
TCCCACTCGACCGGAGGTGTGTTTCGCTCGAGTGGGAGTCGGTCTTCGTG


          *         *         *         *         *
TGGCTGTCAGACCGCACCTACACCTGCCAGGTCACCTATCAAGGTCACAC      800
ACCGACAGTCTGGCGTGGATGTGGACGGTCCAGTGGATAGTTCCAGTGTG


          *         *         *         *         *
CTTTGAGGACAGCACCAAGAAGTGTGCAGATTCCAACCCGAGAGGGGTGA      850
GAAACTCCTGTCGTGGTTCTTCACACGTCTAAGGTTGGGCTCTCCCCACT


          *         *         *         *         *
GCGCCTACCTAAGCCGGCCCAGCCCGTTCGACCTGTTCATCCGCAAGTCG      900
CGCGGATGGATTCGGCCGGGTCGGGCAAGCTGGACAAGTAGGCGTTCAGC


          *         *          *         *         *
CCCACGATCACCTGTCTGGTGGTGGACCTGGCACCCAGCAAGGGGACCGT      950
GGGTGCTAGTGGACAGACCACCACCTGGACCGTGGGTCGTTCCCCTGGCA


          *         *         *         *         *
GAACCTGACCTGGTCCCGGGCCAGTGGGAAGCCTGTGAACCACTCCACCA      1000
CTTGGACTGGACCAGGGCCCGGTCACCCTTCGGACACTTGGTGAGGTGGT
```

2

Figure 1

```
        10        20        30        40        50

        *         *         *         *         *
GAAAGGAGGAGAAGCAGCGCAATGGCACGTTAACCGTCACGTCCACCCTG      1050
CTTTCCTCCTCTTCGTCGCGTTACCGTGCAATTGGCAGTGCAGGTGGGAC


        *         *         *         *         *
CCGGTGGGCACCCGAGACTGGATCGAGGGGGAGACCTACCAGTGCAGGGT      1100
GGCCACCCGTGGGCTCTGACCTAGCTCCCCCTCTGGATGGTCACGTCCCA


        *         *         *         *         *
GACCCACCCCCACCTGCCCAGGGCCCTCATGCGGTCCACGACCAAGACCA      1150
CTGGGTGGGGGTGGACGGGTCCCGGGAGTACGCCAGGTGCTGGTTCTGGT


        *         *         *         *         *
GCGGCCCGCGTGCTGCCCCGGAAGTCTATGCGTTTGCGACGCCGGAGTGG      1200
CGCCGGGCGCACGACGGGGCCTTCAGATACGCAAACGCTGCGGCCTCACC


        *         *         *         *         *
CCGGGGAGCCGGGACAAGCGCACCCTCGCCTGCCTGATCCAGAACTTCAT      1250
GGCCCCTCGGCCCTGTTCGCGTGGGAGCGGACGGACTAGGTCTTGAAGTA


        *         *         *         *         *
GCCTGAGGACATCTCCGGTGCAGTGGCTGCACAACGAGGTGCAGCTCCCCGG    1300
CGGACTCCTGTAGAGCCACGTCACCGACGTGTTGCTCCACGTCGAGGGCC


        *         *         *         *         *
ACGCCCGGCACAGCACGACGCAGCCCCGCAAGACCAAGGGCTCCGGCTTC      1350
TGCGGGCCGTGTCGTGCTGCGTCGGGGCGTTCTGGTTCCCGAGGCCGAAG


        *         *         *         *         *
TTCGTCTTCAGCCGCCTGGAGGTGACCAGGGCCGAATGGGAGCAGAAAGA      1400
AAGCAGAAGTCGGCGGACCTCCACTGGTCCCGGCTTACCCTCGTCTTTCT


        *         *         *         *         *
TGAGTTCATCTGCCGTGCAGTCCATGAGGCAGCGAGCCCCTCACAGACCG      1450
ACTCAAGTAGACGGCACGTCAGGTACTCCGTCGCTCGGGGAGTGTCTGGC


        *         *         *         *         *
TCCAGCGAGCGGTGTCTGTAAATCCCGGTAAATGA
AGGTCGCTCGCCACAGACATTTAGGGCCATTTACT
```

Figure 2

ARG PHE GLN GLY ARG VAL THR MET THR ARG ASP ALA SER PHE SER

THR ALA TYR MET ASP LEU ARG SER LEU ARG SER ASP ASP SER ALA

VAL PHE TYR CYS ALA LYS SER ASP PRO PHE TRP SER ASP TYR TYR

ASN PHE ASP TYR SER TYR THR LEU ASP VAL TRP GLY GLN GLY THR

THR VAL THR VAL SER SER ALA SER THR GLN SER PRO SER VAL PHE

PRO LEU THR ARG CYS CYS LYS ASN ILE PRO SER ASN ALA THR SER

VAL THR LEU GLY CYS LEU ALA THR GLY TYR PHE PRO GLU PRO VAL

MET VAL THR TRP ASP THR GLY SER LEU ASN GLY THR THR MET THR

LEU PRO ALA THR THR LEU THR LEU SER GLY HIS TYR ALA THR ILE

SER LEU LEU THR VAL SER GLY ALA TRP ALA LYS GLN MET PHE THR

CYS ARG VAL ALA HIS THR PRO SER SER THR ASP TRP VAL ASP ASN

LYS THR PHE SER VAL CYS SER ARG ASP PHE THR PRO PRO THR VAL

LYS ILE LEU GLN SER SER CYS ASP GLY GLY GLY HIS PHE PRO PRO

THR ILE GLN LEU LEU CYS LEU VAL SER GLY TYR THR PRO GLY THR

ILE ASN ILE THR TRP LEU GLU ASP GLY GLN VAL MET ASP VAL ASP

LEU SER THR ALA SER THR THR GLN GLU GLY GLU LEU ALA SER THR

GLN SER GLU LEU THR LEU SER GLN LYS HIS TRP LEU SER ASP ARG

THR TYR THR CYS GLN VAL THR TYR GLN GLY HIS THR PHE GLU ASP

SER THR LYS LYS CYS ALA ASP SER ASN PRO ARG GLY VAL SER ALA

TYR LEU SER ARG PRO SER PRO PHE ASP LEU PHE ILE ARG LYS SER

PRO THR ILE THR CYS LEU VAL VAL ASP LEU ALA PRO SER LYS GLY

## Figure 2

THR VAL ASN LEU THR TRP SER ARG ALA SER GLY LYS PRO VAL ASN

HIS SER THR ARG LYS GLU GLU LYS GLN ARG ASN GLY THR LEU THR

VAL THR SER THR LEU PRO VAL GLY THR ARG ASP TRP ILE GLU GLY

GLU THR TYR GLN CYS ARG VAL THR HIS PRO HIS LEU PRO ARG ALA

LEU MET ARG SER THR THR LYS THR SER GLY PRO ARG ALA ALA PRO

GLU VAL TYR ALA PHE ALA THR PRO GLU TRP PRO GLY SER ARG ASP

LYS ARG THR LEU ALA CYS LEU ILE GLN ASN PHE MET PRO GLU ASP

ILE SER VAL GLN TRP LEU HIS ASN GLU VAL GLN LEU PRO ASP ALA

ARG HIS SER THR THR GLN PRO ARG LYS THR LYS GLY SER GLY PHE

PHE VAL PHE SER ARG LEU GLU VAL THR ARG ALA GLU TRP GLU GLN

LYS ASP GLU PHE ILE CYS ARG ALA VAL HIS GLU ALA ALA SER PRO

SER GLN THR VAL GLN ARG ALA VAL SER VAL ASN PRO GLY LYS · —

Figure 3

| Ac | : | Acc I | B | : | Bgl II | S | : | Sma I |
| Al | : | Alu I | Hh | : | Hha I | T | : | Taq I |
| Av | : | Ava II | Hl | : | Hinf I | | | |

100 bp

Figure 4

```
       10        20        30        40        50

       *         *         *         *         *
GGGGGGGGGGGGGGGCGAGATTTCAGGGCAGGGTCACCATGACCAGAGAC      50
CCCCCCCCCCCCCCCGCTCTAAAGTCCCGTCCCAGTGGTACTGGTCTCTG


       *         *         *         *         *
GCGTCCTTCAGTACAGCCTACATGGACCTGAGAAGTCTGAGATCTGACGA     100
CGCAGGAAGTCATGTCGGATGTACCTGGACTCTTCAGACTCTAGACTGCT


       *         *         *         *         *
CTCGGCCGTGTTTTACTGTGCGAAAAGTGACCCTTTTTGGAGTGATTATT     150
GAGCCGGCACAAAATGACACGCTTTTCACTGGGAAAAACCTCACTAATAA


       *         *         *         *         *
ATAACTTTGACTACTCGTACACTTTGGACGTCTGGGGCCAAGGGACCACG     200
TATTGAAACTGATGAGCATGTGAAACCTGCAGACCCCGGTTCCCTGGTGC


       *         *         *         *         *
GTCACCGTCTCCTCAGCCTCCACACAGAGCCCATCCGTCTTCCCCTTGAC     250
CAGTGGCAGAGGAGTCGGAGGTGTGTCTCGGGTAGGCAGAAGGGGAACTG


       *         *         *         *         *
CCGCTGCTGCAAAAACATTCCCTCCAATGCCACCTCCGTGACTCTGGGCT     300
GGCGACGACGTTTTTGTAAGGGAGGTTACGGTGGAGGCACTGAGACCCGA


       *         *         *         *         *
GCCTGGCCACGGGCTACTTCCCGGAGCCGGTGATGGTGACCTGGGACACA     350
CGGACCGGTGCCCGATGAAGGGCCTCGGCCACTACCACTGGACCCTGTGT


       *         *         *         *         *
GGCTCCCTCAACGGGACAACTATGACCTTACCAGCCACCACCCTCACGCT     400
CCGAGGGAGTTGCCCTGTTGATACTGGAATGGTCGGTGGTGGGAGTGCGA


       *         *         *         *         *
CTCTGGTCACTATGCCACCATCAGCTTGCTGACCGTCTCGGGTGCGTGGG     450
GAGACCAGTGATACGGTGGTAGTCGAACGACTGGCAGAGCCCACGCACCC


       *         *         *         *         *
CCAAGCAGATGTTCACCTGCCGTGTGGCACACACTCCATCGTCCACAGAC     500
GGTTCGTCTACAAGTGGACGGCACACCGTGTGTGAGGTAGCAGGTGTCTG
```

7

Figure 4

```
        10        20        30        40        50
         *         *         *         *         *
TGGGTCGACAACAAAACCTTCAGCGTCTGCTCCAGGGACTTCACCCCGCC      550
ACCCAGCTGTTGTTTTGGAAGTCGCAGACGAGGTCCCTGAAGTGGGGCGG


         *         *         *         *         *
CACCGTGAAGATCTTACAGTCGTCCTGCGACGGCGGCGGGCACTTCCCCC      600
GTGGCACTTCTAGAATGTCAGCAGGACGCTGCCGCCGCCCGTGAAGGGGG


         *         *         *         *         *
CGACCATCCAGCTCCTGTGCCTCGTCTCTGGGTACACCCCAGGGACTATC      650
GCTGGTAGGTCGAGGACACGGAGCAGAGACCCATGTGGGGTCCCTGATAG


         *         *         *         *         *
AACATCACCTGGCTGGAGGACGGGCAGGTCATGGACGTGGACTTGTCCAC      700
TTGTAGTGGACCGACCTCCTGCCCGTCCAGTACCTGCACCTGAACAGGTG


         *         *         *         *         *
CGCCTCTACCACGCAGGAGGGTGAGCTGGCCTCCACACAAAGCGAGCTCA      750
GCGGAGATGGTGCGTCCTCCCACTCGACCGGAGGTGTGTTTCGCTCGAGT


         *         *         *         *         *
CCCTCAGCCAGAAGCACTGGCTGTCAGACCGCACCTACACCTGCCAGGTC      800
GGGAGTCGGTCTTCGTGACCGACAGTCTGGCGTGGATGTGGACGGTCCAG


         *         *         *         *         *
ACCTATCAAGGTCACACCTTTGAGGACAGCACCAAGAAGTGTGCAGATTC      850
TGGATAGTTCCAGTGTGGAAACTCCTGTCGTGGTTCTTCACACGTCTAAG


         *         *         *         *         *
CAACCCGAGAGGGGTGAGCGCCTACCTAAGCCGGCCCAGCCCGTTCGACC      900
GTTGGGCTCTCCCCACTCGCGGATGGATTCGGCCGGGTCGGGCAAGCTGG


         *         *         *         *         *
TGTTCATCCGCAAGTCGCCCACGATCACCTGTCTGGTGGTGGACCTGGCA      950
ACAAGTAGGCGTTCAGCGGGTGCTAGTGGACAGACCACCACCTGGACCGT


         *         *         *         *         *
CCCAGCAAGGGGACCGTGAACCTGACCTGGTCCCGGGCCAGTGGGAAGCC      1000
GGGTCGTTCCCCTGGCACTTGGACTGGACCAGGGCCCGGTCACCCTTCGG
```

8

Figure 4

```
          10          20          30          40          50

          *           *           *           *           *
TGTGAACCACTCCACCAGAAAGGAGGAGAAGCAGCGCAATGGCACGTTAA          1050
ACACTTGGTGAGGTGGTCTTTCCTCCTCTTCGTCGCGTTACCGTGCAATT


          *           *           *           *           *
CCGTCACGTCCACCCTGCCGGTGGGCACCCGAGACTGGATCGAGGGGGAG          1100
GGCAGTGCAGGTGGGACGGCCACCCGTGGGCTCTGACCTAGCTCCCCCTC


          *           *           *           *           *
ACCTACCAGTGCAGGGTGACCCACCCCCACCTGCCCAGGGCCCTCATGCG          1150
TGGATGGTCACGTCCCACTGGGTGGGGGTGGACGGGTCCCGGGAGTACGC


          *           *           *           *           *
GTCCACGACCAAGACCAGCGGCCCGCGTGCTGCCCCGGAAGTCTATGCGT          1200
CAGGTGCTGGTTCTGGTCGCCGGGCGCACGACGGGGCCTTCAGATACGCA


          *           *           *           *           *
TTGCGACGCCGGAGTGGCCGGGGAGCCGGGACAAGCGCACCCTCGCCTGC.          1250
AACGCTGCGGCCTCACCGGCCCCTCGGCCCTGTTCGCGTGGGAGCGGACG


          *           *           *           *           *
CTGATCCAGAACTTCATGCCTGAGGACATCTCGGTGCAGTGGCTGCACAA          1300
GACTAGGTCTTGAAGTACGGACTCCTGTAGAGCCACGTCACCGACGTGTT


          *           *           *           *           *
CGAGGTGCAGCTCCCCGGACGCCCGGCACAGCACGACGCAGCCCCGCAAGA          1350
GCTCCACGTCGAGGGCCTGCGGGCCGTGTCGTGCTGCGTCGGGGCGTTCT


          *           *           *           *           *
CCAAGGGCTCCGGCTTCTTCGTCTTCAGCCGCCTGGAGGTGACCAGGGCC          1400
GGTTCCCGAGGCCGAAGAAGCAGAAGTCGGCGGACCTCCACTGGTCCCGG


          *           *           *           *           *
GAATGGGAGCAGAAAGATGAGTTCATCTGCCGTGCAGTCCATGAGGCAGC          1450
CTTACCCTCGTCTTTCTACTCAAGTAGACGGCACGTCAGGTACTCCGTCG  .


          *           *           *           *           *
GAGCCCCTCACAGACCGTCCAGCGAGCGGTGTCTGTAAATCCCGGTAAAT          1500
CTCGGGGAGTGTCTGGCAGGTCGCTCGCCACAGACATTTAGGGCCATTTA
```

9

Figure 4

```
       10         20         30         40         50

       *          *          *          *          *
GACGTACTCCTGCCTCCCTCCCTCCCAGGGCTCCATCCAGCTGTGCAGTG        1550
CTGCATGAGGACGGAGGGAGGGAGGGTCCCGAGGTAGGTCGACACGTCAC


       *          *          *          *          *
GGGAGGACTGGCCAGACCTTCTGTCCACTGTTGCAATGACCCCAGGAAGC        1600
CCCTCCTGACCGGTCTGGAAGACAGGTGACAACGTTACTGGGGTCCTTCG


       *          *          *          *          *
TACCCCCAATAAACTGTGCCTGCTCAGAAAAAAAAAAAAAAAAACCCCCCC       1650
ATGGGGGTTATTTGACACGGACGAGTCTTTTTTTTTTTTTTTTGGGGGGG


       *          *          *          *          *
CCCCCCC
GGGGGGG
```

TOTAL NUMBER OF NUCLEOTIDE PAIRS =        1657

Figure 5

Figure 6

Figure 7

Figure 8

synthetic oligonucleotides

①  d5'CGGTATTACCCGGC3'
②  d5'ATGACAGGAGTAAAAG3'
③  d5'TCATGCCGGGTAATAC3'
④  d5'GATCCTTTTACTCCTG3'

Figure 9

Figure 10

Figure 11